(19) Europäisches Patentamt
European Patent Office
Office européen des brevets

(11) **EP 1 434 854 B1**

(12) **EUROPEAN PATENT SPECIFICATION**

(45) Date of publication and mention
of the grant of the patent:
**27.07.2011 Bulletin 2011/30**

(21) Application number: **02757223.9**

(22) Date of filing: **19.08.2002**

(51) Int Cl.:
*C12N 5/00* (2006.01)      *C12N 15/12* (2006.01)
*C12N 15/63* (2006.01)      *C07K 14/00* (2006.01)
*C07K 16/00* (2006.01)

(86) International application number:
**PCT/US2002/026339**

(87) International publication number:
**WO 2003/031603 (17.04.2003 Gazette 2003/16)**

(54) **COMPOSITIONS USEFUL AS LIGANDS FOR THE FORMYL PEPTIDE RECEPTOR LIKE 1 RECEPTOR AND METHODS OF USE THEREOF**

ALS LIGANDEN FÜR DEN REZEPTOR FORMYLPEPTIDREZEPTOR-ÄHNLICH 1 GEEIGNETE ZUSAMMENSETZUNGEN SOWIE VERFAHREN ZUR VERWENDUNG DAVON

COMPOSITIONS UTILES COMME LIGANDS DU RECEPTEUR DE TYPE RECEPTEUR 1 DES PEPTIDES FORMYLES ET PROCEDES D'UTILISATION DE CELLES-CI

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **09.10.2001 US 328241 P**
**07.05.2002 US 141620**

(43) Date of publication of application:
**07.07.2004 Bulletin 2004/28**

(73) Proprietor: **ChemoCentryx, Inc.**
**Mountain View**
**California 94043 (US)**

(72) Inventors:
• **MIAO, Zhenhua**
**San Jose, CA 95129 (US)**
• **PREMACK, Brett**
**San Francisco, CA 94111 (US)**
• **SCHALL, Thomas, J.**
**Palo Alto, CA 94301 (US)**

(74) Representative: **Baldock, Sharon Claire et al**
**Boult Wade Tennant**
**Verulam Gardens**
**70 Gray's Inn Road**
**London WC1X 8BT (GB)**

(56) References cited:
**WO-A1-96/32481      WO-A1-98/14582**

**US-A- 5 874 211**

• **DATABASE EMBL 13 October 1998 (1998-10-13), "Human MPIF-1 splice variant protein deletion mutant No:2" XP002319948 retrieved from EBI Database accession no. AAW64427**
• **DATABASE EMBL 1 July 1999 (1999-07-01), "hmrp-2a mRNA, complete cds-" XP002319949 retrieved from EBI Database accession no. U58913**
• **YOUN B-S ET AL: "Characterization of CKbeta8 and CKbeta8-1: Two alternatively spliced forms of human beta-chemokine, chemoattractants for neutrophils, monocytes, and lymphocytes, and potent agonists at CC chemokine receptor 1" BLOOD, W.B. SAUNDERS, PHILADELPHIA, VA, US, vol. 91, no. 9, 1 May 1998 (1998-05-01), pages 3118-3126, XP002268293 ISSN: 0006-4971**
• **LE Y ET AL: "PLEIOTROPIC ROLES OF FORMYL PEPTIDE RECEPTORS" CYTOKINE AND GROWTH FACTOR REVIEWS, OXFORD, GB, vol. 12, no. 1, March 2001 (2001-03), pages 91-105, XP001133719 ISSN: 1359-6101**
• **DATABASE EMBL 13 October 1998 (1998-10-13), "Human MIF-1 splice variant protein dletion mutant #1" XP002325770 retrieved from EBI Database accession no. AAW64426**

- SU S B ET AL: "T21 /DP107 A SYNTHETIC LEUCINE ZIPPER-LIKE DOMAIN OF THE HIV-1 ENVELOPE GP41 ATTRACTS AND ACTIVATES HUMAN PHAGOCYTES BY USING G-PROTEIN-COUPLED FORMYL PEPTIDE RECEPTORS" 15 May 1999 (1999-05-15), JOURNAL OF IMMUNOLOGY, THE WILLIAMS AND WILKINS CO. BALTIMORE, US, PAGE(S) 5924-5930 , XP002146726 ISSN: 0022-1767 * abstract * * page 5925, column 1, paragraph 5 - column 2, paragraph 1 * * page 5926, column 2, line 2 - page 5927, column 1, line 1 *

- ELAGOZ ARAM ET AL: "A truncated form of CKbeta8-1 is a potent agonist for human formyl peptide-receptor-like 1 receptor." BRITISH JOURNAL OF PHARMACOLOGY, vol. 141, no. 1, January 2004 (2004-01), pages 37-46, XP002319947 ISSN: 0007-1188

**Description**

FIELD OF THE INVENTION

The invention relates to compositions useful as ligands for the Formyl Peptide Receptor Like 1 receptor and methods of use thereof.

BACKGROUND

Chemokines (chemotactic cytokines) act as molecular beacons for the recruitment and activation of T lymphocytes, neutrophils and macrophages, flagging pathogen battlegrounds. Recruitment of leukocytes, the white blood cells responsible for fighting infections depends on gradients of chemokines. Chemokines are a superfamily of small proteins (8-10 KD) that mediate diverse biological processes including leukocyte trafficking and homing, immunoregulation, hematopoiesis and antiogenesis. To date, 24 chemokines receptors are known. Chemokines play a fundamental role in innate immunity and inflammatory reactions (Baggiolini et al. (*1994*); Baggiolini et al. *(1997);* Rollins (1997).) Four subfamilies of chemokines have been described, based on the distance between the first two conserved cysteine residues: C, CC, CXC, and CX3C. All known chemokines signal through four groups of seven transmembrane receptors which belong to the G protein-coupled receptor and pertussis toxin-sensitive heterotrimeric G proteins of $G_i$ family: XCR, CCR, CXCR and CX3CR. (Murphy et al. (2000)). Extracellular binding events can activate specific signal transduction pathways leading to various responses, such as chemotaxis. In the chemokine system, multiple chemokines may activate a single chemokine receptor; for example, the receptor CCR1 ligates the RANTES (*r*egulated on *a*ctivation *n*ormal *T* cell expressed), MIP-1 $\alpha$ (*m*acrophage inflammatory protein) and MIP-1$\beta$ chemokines. Likewise, a single chemokines .. may activate several receptors (Mantovani (1999)).

Monocytes and neutrophils, which play an important role in the pathogenesis of inflammation and in antigen presentation, respond to chemokines (Lee et al. (2000)). Monocytes express the chemokine receptors CCR1, CCR2, CCR5, CCR8, CXCR2, and CXCR4. (Uguccioni et al. (1995); Weber et al. (2000)). The ligands MIP-1$\alpha$ and Monocyte Chemoattractant Protein 1 (MCP1) have been reported as potent monocyte activators *in vitro.* (Fantuzzi et al. (1999).) Neutrophils are crucial during many acute inflammatory responses, and may also play a role in orienting immunity toward Th1 responses. (Bonecchi et al. (1999).) They mainly respond to some CXC chemokines but do not migrate to most of CC chemokines. Human neutrophils express two high affinity IL-8 receptors, CXCR1 and CXCR2.

The chemokine CK$\beta$8, also known as CCL23; hmrp-2a; myeloid progenitor inhibitor factor 1 (MPIF-1); SCYA23 (current nomenclature and Genome ID system), is a 99-amino acid CC chemokine containing six cysteines. It is constitutively expressed in liver, lung, pancreas, and bone marrow. CK$\beta$8 has chemotactic activity on monocytes, dendritic cells, and resting lymphocytes (Forssmann et al. (1997)) and inhibits colony formation of bone marrow-derived low proliferative potential colony-forming cells. (Patel et al. (1997)). CK$\beta$8-1, an alternative splicing form of CK$\beta$8 that is 116-amino acids in length, has been reported. Both the CK$\beta$8 and CK$\beta$8-1 mature forms have been assigned as ligands for the CCR1 receptor. (Youn et al. (1998)). Cross-desensitization studies in both monocytes and eosinophils indicate that CK$\beta$8-1 binds predominately to the CCR1. Further processing at the $NH_2$-terminus of CK$\beta$8 results in 76 or 75 residue proteins that are significantly more active on CCR1 expressing cells (Macphee et al. (1998), Berkhout et al. (2000)).

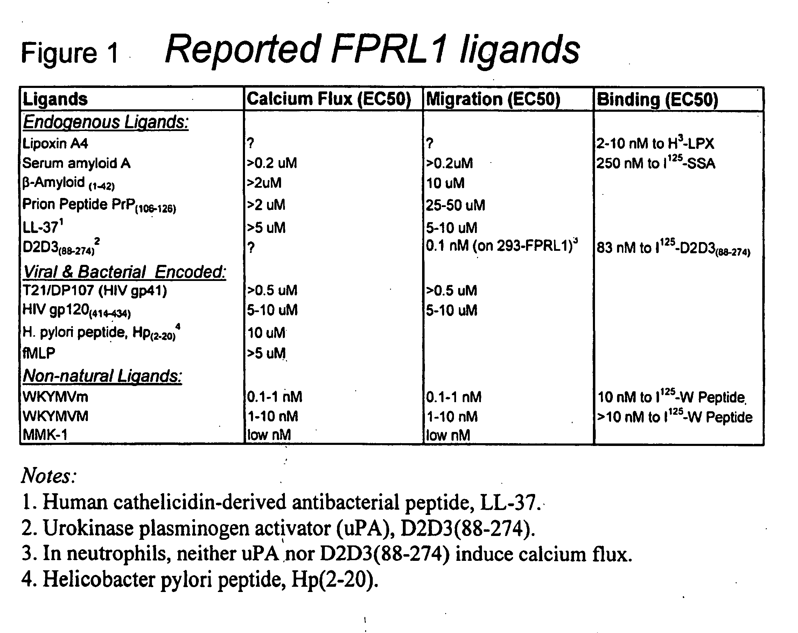

In addition to the chemokine receptors, neutrophils and monocytes also express the G protein-coupled *N*-formyl peptide receptor (FPR) and its homologue *N*-formyl peptide receptor like 1 (FPRL1). Since the ligands for FPRL1 were unknown when it was originally cloned, FPRL1 was initially defined as an orphan receptor. (Bao et al. (1992); Murphy et al. (1992); Ye et al. (1992).) It was assigned as a $LXA_4$ receptor since it binds lipoxin $A_4$ (Fiore et al. (1994).) In addition, several different peptides/proteins have been reported to bind FPRL1 with low affinity (see Figure 1). A serum amyloid A, a protein secreted during the acute phase of inflammation, has been reported as a medial affinity functional ligand (Su et al. (1999)). A $\beta$ amyloid fragment (1-42) and neurotoxic prion peptide 106-126 are also low affinity ligands, indicating that FPRL1 may play a role in neurodegenerative diseases (Le et al. (2001)). Some other low affinity ligands include: peptides derived from HIV envelope proteins (Su et al. (1999), Deng et al. (1999)); and a *Helicobacter pylori* peptide, Hp (2-20). Some synthetic peptides, such as Trp-Lys-Tyr-Met-Val-D-Met- $NH_2$ (WKYMVm) and Trp-Lys-Tyr-Met-Val-Met-$NH_2$ (WKYMVM) ("W peptides 1 and 2"), have been reported as potent ligands for the receptor. (Christophe *et al.* (2001); Baek *et al.* (1996)). However, these non-naturally occurring peptides derived from random hexapeptide libraries have not been shown to be physiologically relevant.

**SUMMARY**

The inventors have discovered that the CKß8-1 truncation variant, CKß8- (25-116), is involved in inflammatory reactions and innate immunity through its role as a functional ligand for the formyl peptide receptor like 1 receptor (FPRL1). In addition, the inventors have discovered an alternatively spliced exon of CKß8-1, named SHAAGtide, and truncated and other variants of SHAAGtide that, along with CKß8-1 (25-116), are functional on both cells that are known to express FPRL1. Functional SHAAGtides generate calcium flux upon receptor-ligand binding in leukocytes and attract monocytes, neutrophils, mature dendritic cells (mDCs), and immature dendritic cells (iDCs).

In one embodiment, the invention encompasses SHAAGtides as well as proteins and peptides comprising SHAAGtides, with the exception of CKß8-1 (25-116). In particular, the present invention provides an isolated protein or polypeptide which modulates FPRL 1 receptor activity comprising a sequence at its N-terminal, the sequence having at least 90% identity to SEQ ID No. 1 over its full length, wherein the protein or polypeptide is not one of SEQ ID Nos. 15 and 16.

In addition, the invention also includes nucleic acids encoding SHAAGtides; nucleic acids encoding proteins and peptides comprising SHAAGtides, antibodies specifically binding SHAAGtides, and fusion proteins comprising SHAAGtides.

In another embodiment, the invention encompasses compositions comprising SHAAGtides or proteins or peptides comprising a SHAAGtide sequence. Such compositions include those suitable for administration to a subject to enhance FPRL1 activity.

In a further embodiment, the invention encompasses kits comprising such compositions. Such kits may be assembled to facilitate administration of, for example, pharmaceutical compositions.

Methods of treating a subject for a disorder comprising modulating an activity of a FPRL1 receptor by administering a compound comprising of a SHAAGtide or proteins or peptides comprising a SHAAGtide sequence are also described.

In a further aspect, the invention encompasses methods and kits useful for the identification of such antagonists are also encompassed by the present invention. Such methods comprise the step of contacting a FPRL1 receptor with a composition comprising a biologically active SHAAGtide, or protein or peptide comprising a SHAAGtide sequence, in the presence of a candidate antigonist molecule. Antagonists to FPRL1 receptor function may be identified as those compounds reducing receptor activity compared to that observed in the absence if the candidate compound.

DESCRIPTION OF THE DRAWINGS

**Figure 1.** Table showing reported FPRL1 endogenous low affinity ligands and non-natural ligands.
**Figure 2.** Figure showing the amino acid sequence alignment of the human CCL23/CKβ8 variants with human CCL15/MIP-1α and CCL3/MIP-1δ.

DETAILED DESCRIPTION

The inventors have discovered that a CKβ8-1 truncation variant, CKβ8-1 (25-116), is a bifunctional ligand for two distinct GPCRs: the chemokine receptor CCR1 and the formyl peptide receptor like 1 receptor (FPRL1). The inventors have also discovered that, in addition to its activity as a CCR1 ligand, CKβ8-1(25-116) is involved in inflammatory reactions and immunity by recruiting monocytes and neutrophils through its role as a functional ligand for FPRL1. CKβ8 attracts cells including monocytes, dendritic cells and resting lymphocytes through OCR1, but lacks the alternatively-spliced exon found in CKβ8-1 (25-116) (SHAAGtide sequence). CKβ8-1(1-116), the alternatively-spliced form of CKβ8 (116 amino acids) is a functional ligand for the CCR1 receptor, as is CKβ8. However, CKβ8-1(1-116) does not exert its functions through the SHAAGtide sequence.

The inventors have also discovered a class of novel peptides (the SHAAGtide peptide and variants of the SHAAGtide peptide - henceforth collectively known as "SHAAGtides"), truncation mutants of the splice exon of the CC chemokine CCL23, CKβ8-1 (25-116), that are surprisingly effective and valuable ligands for the FPRL1 receptor. These peptides produce a calcium flux in leukocytes expressing the FPRL1. In addition, SHAAGtides effectively attract cells including monocytes, neutrophils, mature dendritic cells (mDCs) and immature dendritic cells (iDCs) and other leukocyte subsets. The SHAAGtide peptide (SEQ ID NO:1) and certain SHAAGtide variants, along with their parent chemokine CKβ8-1 (25-116), are functional on both monocytes and neutrophils that are known to express FPRL1. Functional SHAAGtides generate calcium flux upon receptor-ligand binding in leukocytes and attract monocytes, neutrophils, mature dendritic cells (mDCs), and immature Dendritic cells (iDCs) in chemotactic assays. In light of these observations, the SHAAGtides represent cryptic functional peptides that are therefore surprisingly effective as FPRL1 ligands.

The invention encompasses SHAAGtides as well as proteins and peptides comprising SHAAGtides, with the exception of CKβ8-1 (25-116) and CKβ8-1 (1-116). In addition, the invention also includes nucleic acids encoding SHAAGtides, as well as nucleic acids encoding proteins and peptides comprising SHAAGtides, with the exception of nucleic acids encoding CKβ8-1 (25-116)) and CKβ8-1 (1-116). Compositions containing the SHAAGtides as well as proteins and peptides comprising SHAAGtides, including CKβ8-1 (25-116) are also included in the invention. Such compositions include those suitable for administration to a subject to enhance FPRL1 activity. Also included are kits comprising such compositions. Such kits may be assembled to facilitate administration of, for example, pharmaceutical compositions.

Described herein are methods of treating a subject in need of stimulation of inflammatory reactions and innate immunity. Stimulating such activity may benefit subjects suffering from diseases, for example, infectious diseases (and also in vaccination, as described in co-pending patent application "Methods and Compositions for Inducing an Immune Response", filed May 7th, 2002 - attorney reference number 10709/23). Such methods comprise stimulating the FPRL1 receptor by administrating a composition comprising a SHAAGtide, a peptide or protein comprising a SHAAGtide, or other stimulatory molecule.

Also described herein are methods of treating a subject in need of a downregulation of inflammatory reactions and innate immunity. Downregulation of such activity may benefit subjects suffering from diseases including neurodegenerative disorders, such as Alzheimer's disease or Creutzfeldt-Jakob disease. Such methods comprise downregulating the FPRL1 receptor by administrating a composition comprising an antagonist to FPRL1 receptor function.

Methods and kits for the identification of such antagonists are also encompassed by the present invention. Such methods comprise the step of contacting FPRL1 receptor with a composition comprising a biologically active SHAAGtide sequence, a peptide or protein comprising an active SHAAGtide, in the presence of a candidate antagonist molecule. Antagonists to FPRL1 receptor function may be identified as those compounds reducing receptor activity compared to that observed in the absence if the candidate compound. Such methods may be performed in vitro or in vivo. In addition, kits may be assembled to facilitate such in vitro or in vivo tests.

**SHAAGtides and molecules comprising SHAAGtides.**

*SHAAGtide peptides and polypeptides comprising SHAAGtides*

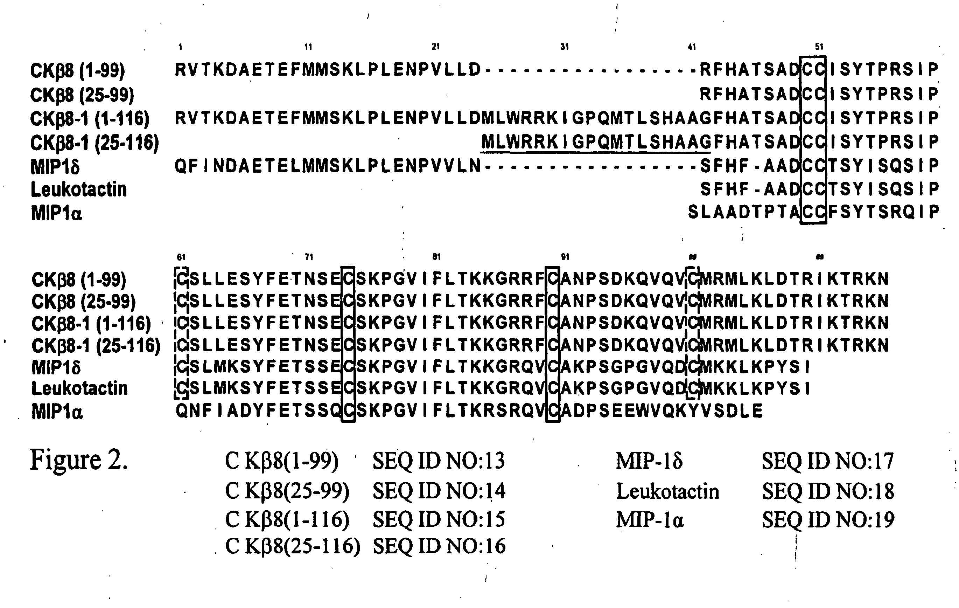

Table 1 shows the SHAAGtide polypeptide sequence (SEQ ID NO:1) and the polypeptide sequences of certain SHAAGtide truncated variants and other variants. Table 2 shows the SHAAGtide polynucleotide sequence (SEQ ID NO:12) and the polynucleotide sequences of SHAAGtide truncated variants and other variants. Table 3 shows the human CKβ8-1(25-116) Nucleotide Sequence (SEQ ID NO:20). Figure 2 shows the amino acid sequence alignment of the human CCL23/CKβ8 variants (CKβ (1-99) - SEQ ID NO: 13; CKβ (25-99) - SEQ ID NO: 14; CKβ (1-116) - SEQ ID NO: 15; CKβ (25-116) - (SEQ ID NO: 16) with human CCL15/MIP-1α (SEQ ID NO: 19); CCL3/MIP-1 S (SEQ ID NO: 17) and Leukotactin (SEQ ID NO: 18). Four conserved cysteine residues are shown in boxes and two additional cysteines, not normally found in the CC chemokine family, are shown in dashed boxes. The alternatively spliced exon of CCL23/CKβ8-1 is shown underlined.

Table 1 SHAAGtide and various truncated and other variants - amino acid sequences.

| SEQ ID NO: | Designation and FPRL1 Activity | Amino acid sequence |
|---|---|---|
| 1 | CCXP1 Native sequence; high activity | Met Leu Trp Arg Arg Lys Ile Gly Pro Gln Met Thr Leu Ser His Ala<br>1           5             10           15<br>Ala Gly<br>    18 |
| 2 | CCXP2 Low activity | Arg Arg Lys Ile Gly Pro Gln Met Thr Leu Ser His Ala Ala Gly<br>1          5            10          15 |
| 3 | CCXP3 High activity | Met Leu Trp Arg Arg Lys Ile Gly Pro Gln Met Thr Leu Ser His<br>1          5            10          15 |
| 4 | CCXP4 Low activity | Ile Gly Pro Gln Met Thr Leu Ser His Ala Ala Gly<br>1          5            10 |
| 5 | CCXP5 Moderate activity | Met Leu Trp Arg Arg Lys Ile Gly Pro Gln Met Thr<br>1          5            10 |
| 6 | CCXP6 high activity | Met Leu Trp Arg Arg Lys Ile Gly Pro Gln Met Thr Leu Ser His Ala<br>1          5            10          15<br>Ala Tyr<br>    18 |
| 7 | CCXP7 Low activity | Trp Arg Arg Lys Ile Gly Pro Gln Met Thr Leu Ser His Ala Ala Gly<br>1          5            10          15 |
| 8 | CCXP8 Moderate activity | Met Leu Trp Arg Arg Lys Ile Gly Pro Gln Met<br>1          5            10 |

| SEQ ID NO: | Designation and FPRL1 Activity | Amino acid sequence |
|---|---|---|
| 9 | CCXP9 Low activity | Trp Arg Arg Lys Ile Gly Pro Gln Met<br>1              5 |
| 10 | CCXP10 Low activity | Trp Arg Arg Lys Ile Gly<br>1              5 |
| 11 | CCXP11 Moderate activity | Leu Trp Arg Arg Lys Ile Gly Pro Gln Met Thr Leu Ser His<br>1          5           10 |

## EP 1 434 854 B1

**Table 2 SHAAGtide and various truncated and other variants - polynucleotide sequences**

| SEQ ID NO: | Polynucleotide sequence | |
|---|---|---|
| 20 | atgctctgga ggagaaagat tggtcctcag atgacccttt ctcatgctgc agga | 54 |
| 21 | aggagaaaga ttggtcctca gatgaccctt tctcatgctg cagga | |
| 22 | atgctctgga ggagaaagat tggtcctcag atgacccttt ctcat | 45 |
| 23 | attggtcctc agatgaccct ttctcatgct gcagga | |
| 24 | atgctctgga ggagaaagat tggtcctcag atgacc | 36 |
| 25 | atgctctgga ggagaaagat tggtcctcag atgacccttt ctcatgctgc atat | 54 |
| 26 | tggaggagaa agattggtcc tcagatgacc ctttctcatg ctgcagga | |
| 27 | atgctctgga ggagaaagat tggtcctcag atg | 33 |
| 28 | tggaggagaa agattggtcc tcagatg | |
| 29 | tggaggagaa agattggt | |
| 30 | ctctggagga gaaagattgg tcctcagatg accctttctc at | 42 |

**Table 3** Human CKβ8-1(25-1 16) Nucleotide Sequence (SEQ ID NO: 12)

```
atgctctgga ggagaaagat tggtcctcag atgacccttt ctcatgctgc aggattccat      60

gctactagtg ctgactgctg catctcctac accccacgaa gcatcccgtg ttcactcctg     120

gagagttact ttgaaacgaa cagcgagtgc tccaagccgg gtgtcatctt cctcaccaag     180

aaggggcgac gtttctgtgc caaccccagt gataagcaag ttcaggtttg catgagaatg     240

ctgaagctgg acacacggat caagaccagg aagaattga                            279
```

*SHAAGtide molecules, derivatives and analogs*

SHAAGtide peptides of the present invention include those molecules listed in Table 1. In addition, various other derivatives of SHAAGtide peptides and nucleotides may be synthesized using standard techniques. Derivatives are nucleic acid sequences or amino acid sequences formed from native compounds either directly or by modification or partial substitution. Analogs are nucleic acid sequences or amino acid sequences that have a structure similar, but not identical, to the native compound but differ from it in respect to certain components or side chains. Analogs may be synthesized or from a different evolutionary origin.

Derivatives and analogs may be full length or other than full length, if the derivative or analog contains a modified nucleic acid or amino acid. For example, SEQ ID NO:3 contains only the first N-terminal 15 amino acids of the SHAAGtide molecule (SEQ ID NO:1). Derivatives or analogs of the *SHAAGtide* nucleic acid or peptide include, molecules comprising regions that are substantially homologous to the *SHAAGtide* nucleic acid or peptide by at least about 70%, 80%, or 95% identity over a nucleic acid or amino acid sequence of identical size or when compared to an aligned sequence in which the alignment is done by a homology algorithm, or whose encoding nucleic acid is capable of hybridizing to a complementary sequence encoding the aforementioned peptide sequences under stringent, moderately stringent, or low stringent conditions. (Ausubel et al., 1987.) A complementary nucleic acid molecule is one that is sufficiently complementary to a sequence, such that hydrogen bonds are formed with few mismatches, forming a stable duplex. "Complementary" refers to Watson-Crick or Hoogsteen base pairing between nucleotides.

The specificity of single stranded DNA to hybridize complementary fragments is determined by the "stringency" of the reaction conditions. Hybridization stringency increases as the propensity to form DNA duplexes decreases. In nucleic acid hybridization reactions, the stringency can be chosen to either favor specific hybridizations (high stringency), which can be used to identify, for example, full-length clones from a library. Less-specific hybridizations (low stringency) can be used to identify related, but not exact, DNA molecules (homologous, but not identical) or segments.

DNA duplexes are stabilized by: (1) the number of complementary base pairs, (2) the type of base pairs, (3) salt concentration (ionic strength) of the reaction mixture, (4) the temperature of the reaction, and (5) the presence of certain organic solvents, such as formamide which decreases DNA duplex stability. In general, the longer the probe, the higher the temperature required for proper annealing. A common approach is to vary the temperature: higher relative temperatures result in more stringent reaction conditions. (Ausubel et al., 1987) provide an excellent explanation of stringency of hybridization reactions.

To hybridize under "stringent conditions" describes hybridization protocols in which nucleotide sequences at least 60% homologous to each other remain hybridized. Generally, stringent conditions are selected to be about 5°C lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% of the probes are occupied at equilibrium.

"Stringent hybridization conditions" conditions enable a probe, primer or oligonucleotide to hybridize only to its target sequence. Stringent conditions are sequence-dependent and will differ. Stringent conditions comprise: (1) low ionic strength and high temperature washes (*e.g.* 15 mM sodium chloride, 1.5 mM sodium citrate, 0.1 % sodium dodecyl sulfate at 50°C); (2) a denaturing agent during hybridization (*e.g.* 50% (v/v) formamide, 0.1% bovine serum albumin, 0.1% Ficoll, 0.1% polyvinylpyrrolidone, 50mM sodium phosphate buffer (pH 6.5; 750 mM sodium chloride, 75 mM sodium citrate at 42°C); or (3) 50% formamide. Washes typically also comprise 5X SSC (0.75 M NaCl, 75 mM sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2.x SSC (sodium chloride/sodium citrate) and 50% formamide at 55°C, followed by a high-stringency wash consisting of 0.1 x SSC containing EDTA at 55°C. Preferably, the conditions are such that sequences at least about 65%, 70%, 75%, 85%, 90%, 95%, 98%, or 99% homologous to each other typically remain hybridized to each other. These conditions are presented as examples and are not meant to be limiting.

"Moderately stringent conditions" use washing solutions and hybridization conditions that are less stringent (Sambrook, 1989), such that a polynucleotide will hybridize to the entire, fragments, derivatives or analogs of SEQ ID NOS:7-12, 14. One example comprises hybridization in 6X SSC, 5X Denhardt's solution, 0.5% SDS and 100 mg/ml denatured salmon sperm DNA at 55°C, followed by one or more washes in 1X SSC, 0.1% SDS at 37°C. The temperature, ionic strength, etc., can be adjusted to accommodate experimental factors such as probe length. Other moderate stringency conditions have been described (Ausubel et al., 1987; Kriegler, 1990).

"Low stringent conditions" use washing solutions and hybridization conditions that are less stringent than those for moderate stringency (Sambrook, 1989), such that a polynucleotide will hybridize to the entire, fragments, derivatives or analogs of SEQ ID NOS:7-12, 14,. A non-limiting example of low stringency hybridization conditions are hybridization in 35% formamide, 5X SSC, 50 mM Tris-HCl (pH 7.5), 5 mM EDTA, 0.02%-PVP, 0.02% Ficoll, 0.2% BSA, 100 mg/ml denatured salmon sperm DNA, 10% (wt/vol) dextran sulfate at 40°C, followed by one or more washes in 2X SSC, 25 mM Tris-HCl (pH 7.4), 5 mM EDTA, and 0.1% SDS at 50°C. Other conditions of low stringency, such as those for cross-species hybridizations are well-described (Ausubel et al., 1987; Kriegler, 1990; Shilo and Weinberg, 1981).

In addition to naturally-occurring allelic variants of SHAAGtide, changes can be introduced by mutation into SEQ ID NO:1 that incur alterations in the amino acid sequences of the encoded SHAAGtide that do not significantly alter SHAAGtide function. For example, an amino acid substitution at the C-terminal amino acid residue has be made in the sequence of SEQ ID NO:6. A "non-essential" amino acid residue is a residue that can be altered from the wild-type sequences of the SHAAGtide without altering biological activity, whereas an "essential" amino acid residue is required for such biological activity. For example, amino acid residues that are conserved among the SHAAGtide of the invention are predicted to be particularly non-amenable to alteration. Amino acids for which conservative substitutions can be made are well known in the art.

Useful conservative substitutions are shown in Table 4, "Preferred substitutions." Conservative substitutions whereby an amino acid of one class is replaced with another amino acid of the same type fall within the scope of the invention so long as the substitution does not materially alter the biological activity of the compound.

Table 4 Preferred substitutions

| Original residue | Exemplary substitutions | Preferred substitutions |
|---|---|---|
| Ala (A) | Val, Leu, Ile | Val |
| Arg (R) | Lys, Gln, Asn | Lys |
| Asn (N) | Gln, His, Lys, Arg | Gln |
| Asp (D) | Glu | Glu |
| Cys (C) | Ser | Ser |
| Gln (Q) | Asn | Asn |
| Glu (E) | Asp | Asp |
| Gly (G) | Pro, Ala - | Ala |
| His (H) | Asn, Gln, Lys, Arg | Arg |
| Ile (1) | Leu, Val, Met, Ala, Phe, Norleucine | Leu |
| Leu (L) | Norleucine, Ile, Val, Met, Ala, Phe | Ile |
| Lys (K) | Arg, Gln, Asn | Arg |
| Met (M) | Leu, Phe, Ile | Leu |
| Phe (F) | Leu, Val, Ile, Ala, Tyr | Leu |
| Pro (P) | Ala | Ala |
| Ser (S) | Thr | Thr |
| Thr (T) | Ser | Ser |
| Trp (W) | Tyr, Phe | Tyr |
| Tyr (Y) | Trp, Phe, Thr, Ser | Phe |
| Val (V) | Ile, Leu, Met, Phe, Ala, Norleucine | Leu, |

Non-conservative substitutions that effect (1) the structure of the polypeptide backbone, such as a β-sheet or α-helical conformation, (2) the charge, (3) hydrophobicity, or (4) the bulk of the side chain of the target site can modify SHAAGtide function, especially when a SHAAGtide sequences comprises a part of a larger polypeptide molecule. Residues are divided into groups based on common side-chain properties as denoted in Table 5. Non-conservative substitutions entail exchanging a member of one of these classes for another class. Substitutions may be introduced into conservative substitution sites or more preferably into non-conserved sites.

Table 5 Amino acid classes

| Class | Amino acids |
|---|---|
| hydrophobic | Norleucine, Met, Ala, Val, Leu, Ile |
| neutral hydrophilic | Cys, Ser, Thr |
| acidic | Asp, Glu |
| basic | Asn, Gln, His, Lys, Arg |
| disrupt chain conformation | Gly, Pro |
| aromatic | Trp, Tyr, Phe |

The variant polypeptides can be made using methods known in the art such as oligonucleotide-mediated (site-directed) mutagenesis, alanine scanning, and PCR mutagenesis. Site-directed mutagenesis (Carter, 1986; Zoller and Smith, 1987), cassette mutagenesis, restriction selection mutagenesis (Wells et al., 1985) or other known techniques can be performed on the cloned DNA to produce the *SHAAGtide* variant DNA (Ausubel et al., 1987; Sambrook, 1989).
An "isolated" or "purified" SHAAGtides of the present invention comprise polypeptides, proteins or biologically active fragments separated and/or recovered from a component of its natural environment Isolated SHAAGtides include those expressed heterologously in genetically engineered cells or expressed *in vitro*. - Contaminant components include materials that would typically interfere with diagnostic or therapeutic uses for the polypeptide. To be substantially isolated, preparations having less than 30% by dry weight of non-SHAAGtide contaminating material (contaminants), more preferably less than 20%, 10% and most preferably less than 5% contaminants.

Polypeptides and fragments of interest can be produced by any method well known in the art, such as by expression via vectors such as bacteria, viruses and eukaryotic cells. In addition, *in vitro* synthesis, such as peptide synthesis, may be also used.

An "active polypeptide or polypeptide fragment" retains a biological and/or an immunological activity similar, but not necessarily identical, to an activity of a SHAAGtide polypeptide shown in Table 1. Immunological activity, in the context of this immediate discussion of the polypeptide per se, and not an actual biological role for SHAAGtide in eliciting or enhancing FPRL1 activity, refers to an aspect of a SHAAGtide polypeptide in that a specific antibody against a SHAAGtide antigenic epitope binds a SHAAGtide. Biological activity refers to a function, either inhibitory or stimulatory, caused by a native SHAAGtide polypeptide. A biological activity of SHAAGtide polypeptide includes, for example, binding to the FPRL1 receptor, or chemotaxis or eliciting calcium flux upon FPRL1 receptor binding. A particular biological assay (see Examples), with or without dose dependency, can be used to determine SHAAGtide activity. A nucleic acid fragment encoding a biologically-active portion of SHAAGtide can be prepared by isolating a polynucleotide sequence that encodes a polypeptide having a SHAAGtide biological activity, expressing the encoded portion of SHAAGtide (*e.g.*, by recombinant expression *in vitro*) and assessing the activity of the encoded portion of SHAAGtide polypeptide.

In general, a SHAAGtide polypeptide variant that preserves SHAAGtide polypeptide-like function and includes any variant in which residues at a particular position in the sequence have been substituted by other amino acids, and further includes the possibility of inserting an additional residue or residues between two residues of the parent protein as well as the possibility of deleting one or more residues from the parent sequence. Any amino acid substitution, insertion, or deletion is encompassed by the invention In favorable circumstances, the substitution is a conservative substitution as defined above.

Table 1 shows that the deletion of amino acids at the C-terminal of the SHAAGtide sequence is less likely to cause a loss of FPRL 1 activity than deletion at the N-terminal (see Example 9). For example, SEQ ID NO:8, consisting of the 11 N-terminal amino acids of the SHAAGtide sequence still retains moderate FPRL1 activity. However, deletion of 3 N-terminal amino acids (SEQ ID NO:2) results in only a low FPRL1 activity. Nevertheless, the deletion of the terminal amino acid at the N-terminal (SEQ ID NO:11) does not result in a complete loss in FRPL 1 activity.

"SHAAGtide variant" means an active SHAAGtide polypeptide having at least: (1) about 80% amino acid sequence identity with a full-length native sequence SHAAGtide polypeptide sequence or (2) any fragment of a full-length SHAAGtide polypeptide sequence. SHAAGtide polypeptide variants include SHAAGtide polypeptides wherein one or more amino acid residues are added or deleted at the N- or C- terminus of the full-length native amino acid sequence, with the exception of those fragments that are identical to CKβ8 and CKβ8-1. A SHAAGtide polypeptide variant will have at least about 90% amino acid sequence identity, more preferably at least about 91%, 92%, 93%, 94%, 95%, 96%, 97%, 98% amino acid sequence identity and most preferably at least about 99% amino acid sequence identity with a full-length native sequence SHAAGtide polypeptide sequence. Ordinarily, SHAAGtide variant polypeptides are at least about 10 amino acids in length, often at least about 20 amino acids in length, more often at least about 30, 40, 50, 60, 70, 80, 90, 100, 150, 200, or 300 amino acids in length, or more.

"Percent (%) amino acid sequence identity" is defined as the percentage of amino acid residues in SHAAGtide that are identical with amino acid residues in a candidate sequence when the two sequences are aligned. To determine % amino acid identity, sequences are aligned and if necessary, gaps are introduced to achieve the maximum % sequence identity; conservative substitutions are not considered as part of the sequence identity. Amino acid sequence alignment procedures to determine percent identity are well known to those of skill in the art. Often publicly available computer software such as BLAST, BLAST2, ALIGN2 or Megalign (DNASTAR) software is used to align peptide sequences.

When amino acid sequences are aligned, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) can be calculated as:

$$\% \text{ amino acid sequence identity} = X/Y \cdot 100$$

where

X is the number of amino acid residues scored as identical matches by the sequence alignment program's or algorithm's alignment of A and B

and

Y is the total number of amino acid residues in B.

If the length of amino acid sequence A is not equal to the length of amino acid sequence B, the % amino acid sequence identity of A to B will not equal the % amino acid sequence identity of B to A.

Fusion polypeptides are useful in expression studies, cell-localization, bioassays, and SHAAGtide purification. A SHAAGtide "chimeric protein" or "fusion protein" comprises SHAAGtide fused to a non-SHAAGtide polypeptide. A non-SHAAGtide polypeptide is not substantially homologous to a SHAAGtide polypeptide. A SHAAGtide fusion protein may include any portion to the entire SHAAGtide, including any number of the biologically active portions. For example, SHAAGtide may be fused to the C-terminus of the GST (glutathione S-transferase) sequences. Such fusion proteins facilitate the purification of recombinant SHAAGtide. In certain host cells, (*e.g.* mammalian), heterologous signal sequences fusions may ameliorate SHAAGtide expression and/or secretion.

Antibodies specific to the SHAAGtide and SHAAGtide variant sequences are also described herein. Methods of producing polyclonal and monoclonal antibodies, including binding fragments (*e.g.*, $F_{(ab)2}$) and single chain versions are well known. Hence, polyclonal or monoclonal antibodies can be prepared by standard techniques.

The chemotactic compositions of the invention contain one or more polynucleotides or polypeptides containing a SHAAGtide sequence. In an embodiment, the composition contains a SHAAGtide that is an isolated or recombinant polynucleotide or polypeptide. In an embodiment, the SHAAGtide(s) is/are the predominant species (*i.e.*, greater than about 50%, more often greater than about 80% by weight of the total of the members of the class of molecule in the composition) of its class (*e.g.*, polypeptide, polynucleotide, lipid, carbohydrate) in the composition. The chemotactic compositions of the invention contain SHAAGtides free of materials normally associated with their *in situ* environment (if naturally occurring).

An isolated SHAAGtide nucleic acid molecule is purified from the setting in which it is found in nature and is separated from at least one contaminant nucleic acid molecule. Isolated *SHAAGtide* molecules are distinguished from the specific *SHAAGtide* molecule, as it exists in cells.

**Use of SHAAGtide compositions in the treatment of disease.**

Herein described are both prophylactic and therapeutic methods of treating a subject at risk of (or susceptible to) a disorder or having a disorder associated with aberrant FPRL1 receptor or FPRL1 ligand activity. Examples include neurodegenerative disorders, such as Alzheimer's Disease.

Diseases or conditions of humans or other species which can be treated with SHAAGtides or proteins or peptides comprising SHAAGtides, or inhibitors or agonists of FPLR1-SHAAGtide interactions, include, but are not limited to, peripheral - chronic inflammation-related diseases, for example: chronic inflammation; thrombosis; atherosclerosis; restenosis; chronic venous insufficiency; recurrent bacterial infections; sepsis; cutaneous infections; renal disease; glomerulonephritis; fibrotic lung disease; allergic disease; IBS; rheumatorid arthritis and acute bronchiolitis. Central nervous system - macroglia and microglia related diseases, for example: neurodegenerative diseases; Alzheimer's disease; Multiple sclerosis; Parkinson's disease; neuroinflammation; HIV-associated neurological diseases; HIV- associated dementia; CNS bacterial infections; brain Toxoplasma gondii; Acanthamoeba infections; Listeria infections; prion diseases; subacute spongiform encephalopathies and macular degeneration may also be treated.

Diseases and disorders that are characterized by increased FPRL1 levels or biological activity may be treated with therapeutics that antagonize (*i.e.*, reduce or inhibit) activity. Antagonists may be administered in a therapeutic or prophylactic manner. Therapeutics that may be used include: (1) molecules comprising inactive SHAAGtide peptides, or analogs, derivatives, fragments or homologs thereof; (2) SHAAGtide antisense nucleic acids (3) antibodies to SHAAGtide peptides, or analogs, derivatives, fragments or homologs thereof or (4) modulators (*i.e.*, inhibitors and antagonists) that antagonize the activity of the FPRL1 receptor.

Diseases and disorders that are characterized by decreased FPRL1 levels or biological activity may be treated with therapeutics that increase (*i.e.*, are agonists to) activity. Therapeutics that up regulate activity may be administered therapeutically or prophylactically. Therapeutics that may be used include peptides, or analogs, derivatives, fragments or homologs thereof; or an agonist that increases bioavailability. Therapeutics that may be used include: (1) molecules comprising SHAAGtide peptides, or analogs, derivatives, fragments or homologs thereof; (2) SHAAGtide nucleic acids; or (3) modulators that agonize the activity of the FPRL1 receptor.

Also herein described is a method for preventing, in a subject, a disease or condition associated with an aberrant FPRL1 receptor expression or activity, by administering an agent that modulates a FPRL1 activity. Subjects at risk for a disease that is caused or contributed to by aberrant FPRL1 activity can be identified by, for example, any or a combination of diagnostic or prognostic assays. Administration of a prophylactic agent can occur prior to the manifestation of symptoms characteristic of the FPRL1 aberrancy, such that a disease or disorder is prevented or, alternatively, delayed in its progression. Depending on the type of FPRL1 aberrancy, for example, a FPRL1 agonist or FPRL1 antagonist can be used to treat the subject The appropriate agent can be determined based on screening assays.

Also described herein are methods of modulating FPRL1 activity for therapeutic purposes. Modulatory methods involve contacting a cell with an agent that modulates one or more of the activities of FPRL1 activity associated with the cell. An agent that modulates FPRL1 activity can be a nucleic acid or a protein, a naturally occurring cognate ligand of FPRL1, a peptide, a SHAAGtide peptidomimetic, or other small molecule. The agent may stimulate FPRL1activity. The agent may inhibit a FPRL1 activity. Modulatory methods can be performed *in vitro* (*e.g.,* by culturing the cell with the agent) or, alternatively, *in vivo (e.g.,* by administering the agent to a subject). For example, the method may involve administering a SHAAGtide or nucleic acid molecule as therapy to compensate for reduced or aberrant FPRL1 or FPRL1 ligand expression or activity.

Stimulation of FPRL1 activity is desirable in situations in which FPRL1, or FPRL1 ligand is abnormally down-regulated and/or in which increased FPRL1, or FPRL1 ligand activity is likely to have a beneficial effect; for example, in treating an infection or in vaccination. Conversely, diminished FPRL1, or FPRL1 ligand activity is desired in conditions in which FPRL1, or FPRL1 ligand activity is abnormally up-regulated and/or in which decreased FPRL1, or FPRL1 ligand activity is likely to have a beneficial effect; for example, in treating chronic inflammation.

Suitable *in vitro* or *in vivo* assays can be performed to determine the effect of a specific therapeutic and whether its administration is indicated for treatment of the affected tissue.

In various specific embodiments, *in vitro* assays may be performed with representative cells of the type(s) involved in the patient's disorder, to determine if a given therapeutic exerts the desired effect upon the cell type(s). Modalities for use in therapy may be tested in suitable animal model systems including, but not limited to rats, mice, chicken, cows, monkeys, rabbits, dogs and the like, prior to testing in human subjects. Similarly, for *in vivo* testing, any of the animal model system known in the art may be used prior to administration to human subjects.

Diseases and conditions associated with inflammation and infection can be treated using the methods of the present invention. The disease or condition is one in which the actions of a FPRL1 ligand on a FPRL1 receptor is to be inhibited or promoted, in order to modulate the immune response.

The compositions may be administered by oral, parenteral (e.g., intramuscular, intraperitoneal, intravenous, ICV, intracisternal injection or infusion, subcutaneous injection, or implant), by inhalation spray, nasal, vaginal, rectal, sublingual, or topical routes of administration and may be formulated, alone or together, in suitable dosage unit formulations containing conventional nontoxic pharmaceutically acceptable carriers, adjuvants and vehicles appropriate for each route of administration. In addition to the treatment of warm-blooded animals such as mice, rats, horses, cattle, sheep, dogs, cats, monkeys, etc., the compositions of the invention are effective for use in humans.

Combined therapy to modulate FPLR1 or FPLR1 ligand activity and thereby prevent and treat infectious diseases or inflammatory disorders and diseases is illustrated by the combination of the compounds of this invention and other compounds which are known for such utilities.

For example, in the treatment or prevention of inflammation, the present compounds may be used in conjunction with an anti-inflammatory or analgesic agent such as an opiate agonist, a lipoxygenase inhibitor, such as an inhibitor of 5-lipoxygenase, a cyclooxygenase inhibitor, such as a cyclooxygenase-2 inhibitor, an interleukin inhibitor, such as TNF$\alpha$, an interleukin-1 inhibitor, an NMDA antagonist, an inhibitor of nitric oxide or an inhibitor of the synthesis of nitric oxide, a non-steroidal anti-inflammatory agent, or a cytokine-suppressing anti-inflammatory agent, for example with a compound such as acetaminophen, aspirin, codeine, fentanyl, ibuprofen, indomethacin, ketorolac, morphine, naproxen, phenacetin, piroxicam, a steroidal analgesic, sufentanyl, sunlindac, tenidap, and the like. Similarly, the instant compounds may be administered with a pain reliever; a potentiator such as caffeine, an H2-antagonist, simethicone, aluminum or magnesium hydroxide; a decongestant such as phenylephrine, phenylpropanolamine, pseudophedrine, oxymetazoline, epinephrine, naphazoline, xylometazoline, propylhexedrine, or levo-desoxy-ephedrine; an anti-itussive such as codeine, hydrocodone, caramiphen, carbetapentane, or dextramethorphan; a steroid; cyclosporin A; methotrexate; IL-10; a diuretic; and a sedating or non-sedating antihistamine.

*Pharmaceutical compositions*

Agonists or antagonists of the FPRL1 receptor can be incorporated into pharmaceutical compositions. Such compositions typically comprise the agonists or antagonists and a pharmaceutically acceptable carrier. A "pharmaceutically acceptable carrier" includes any and all solvents, dispersion media, coatings, antibacterial and antifungal agents, isotonic and absorption delaying agents, and the like, compatible with pharmaceutical administration (Gennaro (2000)). Preferred examples of such carriers or diluents include, but are not limited to, water, saline, Finger's solutions, dextrose solution, and 5% human serum albumin. Liposomes and non-aqueous vehicles such as fixed oils may also be used. Except when a conventional media or agent is incompatible with an active compound, use of these compositions is contemplated. Supplementary active compounds can also be incorporated into the compositions.

A pharmaceutical composition of the agonist or antagonist is formulated to be compatible with its intended route of administration, including intravenous, intradermal, subcutaneous, oral (*e.g.*, inhalation), transdermal (*i.e.*, topical), transmucosal, and rectal administration. Solutions or suspensions used for parenteral, intradermal, or subcutaneous application can include: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampules, disposable syringes or multiple dose vials made of glass or plastic.

Pharmaceutical compositions suitable for injection include sterile aqueous solutions (where water soluble) or dispersions and sterile powders for the extemporaneous preparation of sterile injectable solutions or dispersion. For intravenous administration, suitable carriers include physiological saline, bacteriostatic water, CREMOPHOR EL™ (BASF, Parsippany, N.J.) or phosphate buffered saline (PBS). In all cases, the composition must be sterile and should be fluid so as to be administered using a syringe. Such compositions should be stable during manufacture and storage and must be preserved against contamination from microorganisms such as bacteria and fungi. The carrier can be a solvent or dispersion medium containing, for example, water, ethanol, polyol (such as glycerol, propylene glycol, and liquid polyethylene glycol), and suitable mixtures. Proper fluidity can be maintained, for example, by using a coating such as lecithin, by maintaining the required particle size in the case of dispersion and by using surfactants. Various antibacterial and antifungal agents, for example, parabens, chlorobutanol, phenol, ascorbic acid, and thimerosal, can contain microorganism contamination. Isotonic agents, for example, sugars, polyalcohols such as manitol, sorbitol, and sodium chloride can be included in the composition. Compositions that can delay absorption include agents such as aluminum monostearate and gelatin.

Sterile injectable solutions can be prepared by incorporating the active compound in the required amount in an appropriate solvent with one or a combination of ingredients as required, followed by sterilization. Generally, dispersions are prepared by incorporating the active compound into a sterile vehicle that contains a - basic dispersion medium, and the other required ingredients. Sterile powders for the preparation of sterile injectable solutions, methods of preparation include vacuum drying and freeze-drying that yield a powder containing the active ingredient and any desired ingredient from a sterile solutions.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally. Pharmaceutically compatible binding agents, and/or adjuvant materials can be included. Tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, PRIMOGEL, or corn starch; a lubricant such as magnesium stearate or STEROTES; a glidant such as colloidal silicon dioxide; a sweetening agent such as sucrose or saccharin; or a flavoring agent such as peppermint, methyl salicylate, or orange flavoring.

For administration by inhalation, the compounds are delivered as an aerosol spray from a nebulizer or a pressurized container that contains a suitable propellant, *e.g.*, a gas such as carbon dioxide.

Systemic administration can also be transmucosal or transdermal. For transmucosal or transdermal administration, penetrants that can permeate the target barrier(s) are selected. Transmucosal penetrants include, detergents, bile salts, and fusidic acid derivatives. Nasal sprays or suppositories can be used for transmucosal administration. For transdermal administration, the active compounds are formulated into ointments, salves, gels, or creams.

The compounds can also be prepared in the form of suppositories (*e.g.*, with bases such as cocoa butter and other glycerides) or retention enemas for rectal delivery.

The active compounds can be prepared with carriers that protect the compound against rapid elimination from the body, such as a controlled release formulation, including implants and microencapsulated delivery systems. Biodegradable or biocompatible polymers can be used, such as ethylene vinyl acetate, polyanhydrides, polyglycolic acid, collagen, polyorthoesters, and polylactic acid. Polyethylene glycols, e.g. *PEG,* are also good carriers. Such materials can be obtained commercially from ALZA Corporation (Mountain View, CA) and NOVA Pharmaceuticals, Inc. (Lake Elsinore, CA), or prepared by one of skill in the art. Liposomal suspensions can also be used as pharmaceutically acceptable carriers. These can be prepared according to methods known to those skilled in the art, such as in (Eppstein et al. US Patent No. 4,522,811. 1985).

Oral formulations or parenteral compositions in unit dosage form can be created to facilitate administration and dosage uniformity. Unit dosage form refers to physically discrete units suited as single dosages for the subject to be treated, containing a therapeutically effective quantity of active compound in association with the required pharmaceutical carrier. The specification for the unit dosage forms of the invention are dictated by, and directly dependent on, the unique characteristics of the active compound and the particular desired therapeutic effect, and the inherent limitations of compounding the active compound.

The nucleic acid molecules of *SHAAGtide* can be inserted into vectors and used as gene therapy vectors. Gene therapy vectors can be delivered to a subject by, for example, intravenous injection, local administration (Nabel and Nabel, US Patent No. 5,328,470, 1994), or by stereotactic injection (Chen et al. (1994)). The pharmaceutical preparation of a gene therapy vector can include an acceptable diluent, or can comprise a slow release matrix in which the gene delivery vehicle is imbedded. Alternatively, where the complete gene delivery vector can be produced intact from recombinant cells, *e.g.*, retroviral vectors, the pharmaceutical preparation can include one or more cells that produce the gene delivery system.

In one aspect, the SHAAGtide is delivered as DNA such that the polypeptides are generated *in situ*. In one embodiment, the DNA is "naked," as described, for example, in Ulmer *et al.* (1993) and reviewed by Cohen, (1993). The uptake of naked DNA may be increased by coating the DNA onto a carrier, *e.g.* biodegradable beads, which is efficiently transported into the cells. In such vaccines, the DNA may be present within any of a variety of delivery systems known to those of ordinary skill in the art, including nucleic acid expression systems, bacterial and viral expression systems.

Vectors, used to shuttle genetic material from organism to organism, can be divided into two general classes: Cloning vectors are replicating plasmid or phage with regions that are non-essential for propagation in an appropriate host cell and into which foreign DNA can be inserted; the foreign DNA is replicated and propagated as if it were a component of the vector. An expression vector (such as a plasmid, yeast, or animal virus genome) is used to introduce foreign genetic material into a host cell or tissue in order to transcribe and translate the foreign DNA, such as SHAAGtide. In expression vectors, the introduced DNA is operably-linked to elements such as promoters that signal to the host cell to transcribe the inserted DNA. Some promoters are exceptionally useful, such as inducible promoters that control gene transcription in response to specific factors. Operably-linking a SHAAGtide polynucleotide to an inducible promoter can control the expression of a SHAAGtide polypeptide or fragments. Examples of classic inducible promoters include those that are responsive to $\alpha$-interferon, heat shock, heavy metal ions, and steroids such as glucocorticoids (Kaufman, 1990. Methods Enzymol 185: 487-511.) and tetracycline. Other desirable inducible promoters include those that are not endogenous to the cells in which the construct is being introduced, but, however, are responsive in those cells when the induction agent is exogenously supplied. In general, useful expression vectors are often plasmids. However, other forms of expression vectors, such as viral vectors (*e.g.*, replication defective retroviruses, adenoviruses and adeno-associated viruses) are contemplated.

Vector choice is dictated by the organism or cells being used and the desired fate of the vector. Vectors may replicate once in the target cells, or may be "suicide" vectors. In general, vectors comprise signal sequences, origins of replication, marker genes, enhancer elements, promoters, and transcription termination sequences.

The pharmaceutical composition may further comprise other therapeutically active compounds as noted herein which are usually applied in the treatment of FPRL1-related conditions.

In the treatment or prevention of conditions which require FPRL1 modulation an appropriate dosage level of an agonist or antagonist will generally be about 0.01 to 500 mg per kg patient body weight per day which can be administered in single or multiple doses. Preferably, the dosage level will be about 0.1 to about 250 mg/kg per day; more preferably about 0.5 to about 100 mg/kg per day. A suitable dosage level may be about 0.01 to 250 mg/kg per day, about 0.05 to 100 mg/kg per day, or about 0.1 to 50 mg/kg per day. Within this range the dosage may be 0.05 to 0.5, 0.5 to 5 or 5 to 50 mg/kg per day. For oral administration, the compositions are preferably provided in the form of tablets containing 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0, and 1000.0 milligrams of the active ingredient for the symptomatic adjustment of the dosage to the patient to be treated. The compounds may be administered on a regimen of 1 to 4 times per day, preferably once or twice per day.

However, the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

*Kits*

In an aspect, the invention provides kits containing one or more of the following in a package or container: (1) a biologically active composition of the invention or an FPRL1 antagonist; (2) a pharmaceutically acceptable adjuvant or excipient; (3) a vehicle for administration, such as a syringe; (4) instructions for administration. Embodiments in which two or more of components (1) - (4) are found in the same container are also contemplated.

When a kit is supplied, the different components of the composition may be packaged in separate containers and admixed immediately before use. Such packaging of the components separately may permit long-term storage without losing the active components' functions.

The reagents included in the kits can be supplied in containers of any sort such that the life of the different components are preserved and are not adsorbed or altered by the materials of the container. For example, sealed glass ampules may contain lyophilized SHAAGtide polypeptide or polynucleotide, or buffers that have been packaged under a neutral, non-reacting gas, such as nitrogen. Ampules may consist of any suitable material, such as glass, organic polymers, such as polycarbonate, polystyrene, *etc.*; ceramic, metal or any other material typically employed to hold similar reagents. Other examples of suitable containers include simple bottles that may be fabricated from similar substances as ampules, and envelopes, that may comprise foil-lined interiors, such as aluminum or an alloy. Other containers include test tubes, vials, flasks, bottles, syringes, or the like. Containers may have a sterile access port, such as a bottle having a stopper that can be pierced by a hypodermic injection needle. Other containers may have two compartments that are separated by a readily removable membrane that upon removal permits the components to be mixed. Removable membranes may be glass, plastic, rubber, *etc.*

Kits may also be supplied with instructional materials. Instructions may be printed on paper or other substrate, and/or may be supplied as an electronic-readable medium, such as a floppy disc, CD-ROM, DVD-ROM, Zip disc, videotape, audiotape, *etc.* Detailed instructions may not be physically associated with the kit; instead, a user may be directed to an internet web site specified by the manufacturer or distributor of the kit, or supplied as electronic mail.

### *Screening and detection methods*

SHAAGtides (and SHAAGtide nucleotides used to express SHAAGtides) can be used as reagents in methods to screen for compounds that modulate FPRL1 I receptor activity. Such compounds may be useful in treating disorders characterized by insufficient or excessive production of FPRL1 receptor or FPRL1 receptor ligand, or production of FPRL1 receptor or FPRL1 receptor ligand forms that have aberrant activity compared to wild-type molecules. In general, such compounds may be used to modulate biological functions that involve FPRL1 receptor/ FPRL1 receptor ligand.

The invention provides methods (screening assays) for identifying modalities, *i.e.*, candidate or test compounds or agents (*e.g.,* peptides, peptidomimetics, small molecules or other drugs), foods, combinations thereof, *etc.,* that affect the FPRL1 receptor or FPRL1 receptor ligand. This may be a stimulatory or inhibitory effect. The invention also includes compounds identified in such screening assays.

Testing for compounds that increase or decrease FPRL I receptor activity in response to or independent of a ligand is desirable. A compound may modulate FPRL1 receptor activity by increasing or decreasing the activity of FPRL1 receptor itself (agonists and antagonists).

Test compounds can be obtained using any of the numerous approaches in combinatorial library methods, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptides, while the other four approaches encompass peptide, non-peptide oligomer or small molecule libraries of compounds (Lam, 1997).

A "small molecule" refers to a composition that has a molecular weight of less than about 5 kD and more preferably less than about 4 kD, and most preferably less than 0.6 kD. Small molecules can be, nucleic acids, peptides, polypeptides, peptidomimetics, carbohydrates, lipids or other organic or inorganic molecules. Libraries of chemical and/or biological mixtures, such as fungal, bacterial, or algal extracts, are known in the art and can be screened with any of the assays of the invention. Examples of methods for the synthesis of molecular libraries have been described (Carell et al., 1994a; Carell et al., 1994b; Cho et al., 1993; DeWitt et al., 1993; Gallop et al., 1994; Zuckermann et al., 1994).

Libraries of compounds may be presented in solution (Houghten et al., 1992) or on beads (Lam et al., 1991), on chips (Fodor et al., 1993), bacteria, spores (Ladner et al., US Patent No. 5,223,409, 1993), plasmids (Cull et al., 1992) or on phage (Cwirla et al., 1990; Devlin et al., 1990; Felici et al., 1991; Ladner et al., US Patent No. 5,223,409, 1993; Scott and Smith, 1990).

Many assays for screening candidate or test compounds that bind to or modulate the activity of the FPRL1 receptor are available. A cell-free assay comprises, for example, contacting the FPRL1 receptor or biologically-active fragment with a SHAAGtide compound that binds the FPRL1 receptor to form an assay mixture, contacting the assay mixture with a test compound, and determining the ability of the test compound to interact with the FPRL1 receptor, where determining the ability of the test compound to interact with the FPRL1 receptor comprises determining the ability of the FPRL1 receptor to preferentially bind to or modulate the activity of the test compound. Cell-based assays include, for example, the calcium flux assays, binding assays and cellular migation assays discussed in the examples.

Immobilizing either a molecule containing a SHAAGtide sequence or one of its partner molecules (such as FPRL1) can facilitate separation of complexed from uncomplexed forms of one or both of the proteins, as well as to accommodate high throughput assays. Binding of a test compound to a SHAAGtide molecule or a FPRL1 receptor molecule, or interaction of SHAAGtide molecule with a FPRL1 receptor molecule in the presence and absence of a candidate compound, can be accomplished in any vessel suitable for containing the reactants, such as microtiter plates, test tubes, and micro-centrifuge tubes. A fusion protein can be provided that adds a domain that allows one or both of the proteins to be bound to a matrix. For example, GST (glutathione S-transferase)-SHAAGtide fusion proteins or GST-target fusion proteins can be adsorbed onto glutathione sepharose beads (SIGMA Chemical, St. Louis, MO) or glutathione derivatized microtiter plates that are then combined with the test compound or the test compound and either the non-adsorbed FPRL1 receptor or SHAAGtide molecule, and the mixture is incubated under conditions conducive to complex formation (*e.g.*, at physiological conditions for salt and pH). Following incubation, the beads or microtiter plate wells are washed to remove any unbound components, the matrix immobilized in the case of beads, complex determined either directly or indirectly. Alternatively, the complexes can be dissociated from the matrix, and the level of SHAAGtide binding or activity determined using standard techniques.

Other techniques for immobilizing proteins on matrices can also be used in screening assays. See, for example co-pending United States Patent Application Serial Number 09/721, 902. Either a SHAAGtide molecule or a FPRL1 receptor molecule can be immobilized using biotin-avidin or biotin-streptavidin systems. Biotinylation can be accomplished using many reagents, such as biotin-NHS (N-hydroxy-succinimide; PIERCE Chemicals, Rockford, IL), and immobilized in wells of streptavidin-coated 96 well plates (PIERCE Chemical). Alternatively, antibodies or antibody fragments reactive with SHAAGtide molecules or FPRL1 receptor molecules but which do not interfere with binding of the SHAAGtide to the FPRL1 receptor molecule can be derivatized to the wells of the plate, and FPRL1 receptor molecule or SHAAGtide trapped in the wells by antibody conjugation. Methods for detecting such complexes, in addition to those described for the GST-immobilized complexes, include immunodetection of complexes using antibodies reactive with FPRL1 receptor molecules or SHAAGtide molecules, as well as enzyme-linked assays that rely on detecting an enzymatic activity associated with the FPRL1 receptor molecules or SHAAGtide molecules.

To demonstrate that the compounds are antagonists of the FPRL1 receptor, one can determine if they inhibit the activity of a SHAAGtide on the receptor. Preferably such compounds have the at least one of the following characteristics:

(1) potently inhibit binding of a SHAAGtide or a molecule comprising a SHAAGtide sequence to the FPRL1 receptor;
(2) significant inhibition of the $Ca^{2+}$ response of a SHAAGtide or a molecule comprising a SHAAGtide binding to FPRL1;
(3) limited non-specific $Ca^{2+}$ response; or
(4) inhibition of chemotactic activity.

Standard *in vitro* binding assays may be employed to demonstrate the affinity of the compounds for the FPRL1 receptor (thereby inhibiting the activity of a SHAAGtide by competitive interaction with the receptor). See examples below. Preferably, the active compounds exhibit an $IC_{50}$ value of <10 $\mu$M, more preferably <5 $\mu$M, most preferably <1 $\mu$M.

Compounds that inhibit the activity of SHAAGtide affect intracellular $Ca^{2+}$ concentration in SHAAGtide stimulated cells. Ligand binding to the FPRL1 receptor results in G-protein induced activation of phospholipase C, which leads to the conversion of phosphatidyl inositol phosphate into inositol phosphate and diacylglycerol. Inositol phosphate in turn binds to a receptor located at intracellular sites to release $Ca^{2+}$ into the cytoplasm. In addition to $Ca^{2+}$ concentration increases due to release from intracellular stores, binding of inositol phosphate to its receptor leads to an increased flux of extracellular calcium across the membrane and into the cell. Other G-protein signaling pathways may be involved.

Thus, the activation of the FPRL1 receptor by a SHAAGtide, and, subsequently, inhibition of the activation by the compounds of the invention can be determined by assaying for an increase in free intracellular $Ca^{2+}$ levels. Typically, this can be achieved by the use of calcium-sensitive fluorescent probes such as quin-2, fura-2 and indo-1. The affect of the active compounds to block the $Ca^{2+}$ response depends on the amount of active compound and chemokine present. Generally, when 10 nM of chemokine is present, 10 $\mu$M of active compound should produce 20 to 100% inhibition of the $Ca^{2+}$ response.

To determine whether the active compound produces a non-specific Ca2+ response, cells bearing multiple receptors, including the receptor to which the active compound is targeted, are incubated with compound. Cells are then stimulated with a ligand to the target receptor and sequentially followed by stimulation with ligands to other receptors found on the sample cells. A comparable response of non-target receptors to ligand in the presence or absence of compound indicates that the active compound is specific for the target receptor.

To determine chemotaxis, any cell migration assay format may be used, such as the ChemoTx® system (NeuroProbe, Rockville, MD) or any other suitable device or system (Bacon et al., 1988; Penfold et al., 1999). In brief, these cell migration assays work as follows. After harvesting and preparing the cells bearing the active target chemokine receptor, the cells are mixed with candidate antagonists. The mixture is placed into the upper chamber of the cell migration apparatus. To the lower chamber, a stimulatory concentration of chemokine ligand is added. The migration assay is then executed, terminated, and cell migration assessed.

The inventors have shown SHAAGtide activity on the FPRL1 receptor expressed on monocytes, neutrophils, Immature Dendritic Cells and Mature Dendritic Cells. Hence, such cells may be used in *in vitro* assay methods. Enriched or substantially purified cell populations can be used in *in vitro* chemotaxis assays. These cell populations can be prepared by a variety of methods known in the art depending on the specific cell-type desired. Typically, substantially purified cell populations are prepared by culture under specific conditions, by physical characteristics such as behavior in a density gradient, by sorting according to characteristic markers (*e.g.*, by fluorescence activated cell sorting (FACS) using antibodies (preferably monoclonal antibodies) to cell-surface proteins, immunoprecipitation), or other methods.

Cells can be identified by histology (see, *e.g.*, Luna, 1968), by immunological staining and similar methods (see, *e.g.*, Harlow et al. 1998; Coligan *et al.,* 1991. Methods for preparing substantially purified cell compositions for use in *in vitro* chemotaxis assays are briefly described *infra* and in the Examples. However, the invention does not require that any particular purification method be used, so long as the desired cells are obtained; many variations and alternative methods are known to those of skill in the art. Further, many other purification and detection methods, including methods suitable for cells not specifically listed herein, are known in the art or can be easily developed. Further, cloned cell lines derived from immune system tissues can be used in the chemotaxis assays described herein, if desired. General immunological, purification and cell culture methods are described in Coligan *et al.* (1991), including supplements through 1999. Unless otherwise specified, cells in culture are incubated at 37˚C in 5% $CO_2$.

Suitable methods for monocyte purification are found in Bender *et al.,* 1996. (also see U.S. Pat. No. 5,994,126). Briefly, monocytes are isolated from PBMC by depleting T cells using immobilized antibodies against a pan T cell surface marker CD2. Conveniently, a commercially available source of CD2 antibodies attached to magnetic beads (Dynal; Lake Success, NY) is used. PBMC isolated from a buffy coat (typically 35 mls containing 400 x $10^6$ PMBC) by conventional Ficoll gradient centrifugation methods are resuspended in MACS buffer (DPBS (HyClone; Logan, UT) with 1% BSA (Sigma)) at 20 x $10^6$ cells per ml. DPBS is Dulbecco's Phosphate Buffered Saline ($CaCl_2$ (0.1g/l), KCl (0.2g/l), $KH_2PO_4$ (0.2g/l), $MgCl_2$-$6H_2O$ (0.1g/l), NaCl (8.0g/l), $Na_2HPO4$ (2.16g/l)). An appropriate amount of immobilized CD2 + magnetic beads (typically 10 $\mu$l per $10^6$ cells) are added to the cells. The mixture is incubated for 15 minutes at 4˚C with gentle rotation. The magnetically tagged T cells are removed from the unlabeled cells on a magnetic cell sorter (Dynal) according to the manufacturer's protocols. The unlabeled cells contain primarily monocytes and B cells.

B cells in the above preparation are removed by taking advantage of differential adhesion properties. Briefly, PBMC depleted of T cells are allowed to adhere to the plastic of a T-175 tissue culture flask (100 x $10^6$ cells/ flask; Costar; Acton, MA) for 3 hours at 37˚C. Non-adherent cells (comprising largely B cells) are aspirated. To completely remove non-adherent cells, the flasks are rinsed 3 more times with DPBS. The resulting cells are largely enriched (*i.e.*, > 90%) for monocytes.

Monocytes can also be isolated by positive selection of CD14 antigen. Briefly, PBMC isolated from peripheral blood, such as a buffy coat, by standard Ficoll gradient centrifugation methods are resuspended in MACS buffer at 1 $\times$ $10^6$ cells/ml. Immobilized antibodies against the CD14 surface antigen, such as CD14+ magnetic microbeads (Milteyni) are added (1 $\mu$l of beads per $1\times10^6$ cells) and the mixture is incubated at 4˚C for 15 minutes. Monocytes are separated from the other cell populations by passing the mixture through a positive selection column on a magnetic cell sorter (Miltenyi Biotech; Auburn, CA) according to manufacturers protocol. Monocytes that are retained on the column are eluted with MACS buffer after the column is removed from the MACS apparatus. Cells are then pelleted by centrifugation and resuspended in RMPI plus 10% FCS media at $10^6$ cells per ml. Monocytes isolated by this method are cultured essentially the same way as those isolated by the CD2+ depletion method.

Suitable methods for purification of dendritic cells, including separate mature and immature populations, are known in the art. Substantially purified dendritic cells (including subpopulations of mature or immature cells) can be prepared by selective *in vitro* culture conditions.

Dendritic cells are widely distributed in all tissues that have contact with potential pathogens (*e.g.*, skin, gastrointestinal and respiratory tracts, and T cell-rich areas of the secondary lymphoid tissues). In the skin and upper respiratory tract they form a lattice of highly arborised cells (called Langerhans cells in the skin). After capturing antigen, dendritic cells in the peripheral tissues such as the skin and gut, traffic via the draining lymphatics to the T cell areas of lymph nodes where they present the internalized antigen. Immature dendritic cells function to take up and process antigens. During subsequent migration to the draining lymph node, the DC matures. The mature dendritic cells functions as the key APC to initiate immune responses by inducing the proliferation of pathogen specific cytotoxic and helper T cells.

Substantially pure populations of dendritic cells can be produced by *in vitro* culture, *infra*). In addition, there are marked changes in expression of chemokine receptors during dendritic cell maturation which can be used to identify cell stage (Campbell *et al.* 1998; Chan *et al.* 1999; Dieu *et al.* 1998; Kellermann *et al.* 1999). For example, immature dendritic cells express predominately CCR1, CCR5, and CXCR4. Upon maturation, these receptors, with the exception of CXCR4, are down regulated.

In culture, immature forms of dendritic cells undergo maturation thought to be analogous to the events during migration of dendritic cells from the point of antigen contact until to the secondary lymphoid tissues. Human or macaque dendritic cells of various developmental stages can be generated in culture, from CD14$^+$ blood progenitors using specific cytokines. A separate lineage of dendritic cells can be differentiated from CD34+ precursor cells from cord blood or bone marrow. In one embodiment of the invention, subpopulations of dendritic cells are generated for *in vitro* assays for identification of chemotactic compositions (*i.e.* to assess chemotaxin potency and selectivity against defined DC subtypes). Exemplary subpopulations of dendritic cells are: (1) immature peripheral blood monocyte derived cells; (2) mature peripheral blood monocyte derived cells, and (3) cells derived from CD34+ precursors. Subpopulations are isolated or produced by a variety of methods known in the art. For example, immature and mature dendritic cells from PBMCs are produced according to Bender *et al. supra.*

Briefly, PBMCs are depleted of T cells using immobilized antibodies against the cell surface marker CD2 (present on all T cells). Commercially available CD2+ dynabeads (Dynal) can be used according to manufacturer's protocol. The T-cell depleted mixture is separated into adherent versus non-adherent fractions by incubating the cells on tissue culture grade plastic for 3 hours at 37˚C. Non-adherent cells are gently removed, and adherent cells (generally CD14$^+$ monocytes) are placed in culture media (*e.g.*, RMPI + 10% FCS) supplemented with 1000 U/mL each of GM-CSF and IL-4 (R&D Systems, Minneapolis, MN) ("Day 1"). Between days 3-7 the cells begin to display a veiled morphology, and cytokines are replenished on days 2, 4, and 6, at which time the cells can be harvested as immature dendritic cells. In one embodiment, cells of this *in vitro* stage are isolated and used in the assay. Approximately $10 \times 10^6$ dendritic cells are typically obtained from $400 \times 10^6$ PBMCs.

Day 7 immature dendritic cells exhibit typical dendritic cell morphology, with elongated cell body and many processes. The size of the cells increase significantly compared to the precursor monocytes. Immature dendritic cells can be characterized phenotypically by monitoring their expression of cell surface markers.

Immature dendritic cells (generated from peripheral blood monocytes or from bone marrow derived CD34+ precursors) can be further activated and differentiated to become mature dendritic cells. Two methods are primarily used: MCM (macrophage conditioned medium) and double-stranded RNA-ploy (I:C) stimulation (Cella et al, 1999; Verdijk *et al.* 1999).

In the MCM method, day 6 immature dendritic cells are harvested by centrifugation and resuspended in at $10^6$ cells/ml in maturation medium (*e.g.*, MCM diluted (up to 1:1 with RPMI containing 10% FCS). GM-CSF (1000 U/ml) and IL-4 (1000 U/ml) are added. Cells are cultured for three more days, without further addition of GM-CSF (1000 U/ml) and IL-4. Day 9 cells are used as mature dendritic cells.

In the poly (I:C) method, day 6 immature dendritic cells are harvested and resuspended in the standard culture medium (RPMI plus 10% FCS) supplemented with 20 μg/ml of poly (I:C) (Sigma), 1000 U/ml of GM-CSF and IL-4. Cells are cultured for another three days without additional cytokines. Day 9 cells are used as mature dendritic cells.

Mature dendritic cells generated by these two different methods exhibit phenotypic and functional properties distinct from those of immature dendritic cells or the precursor monocytes. Mature dendritic cells from each preparation are thoroughly characterized by FACS to ensure that the desirable cell types are obtained.

Notably, generated mature dendritic cells express significantly higher level of MHC class II on the cell surface than immature cells. Expression of CD80, CD83 and CD86 are also up-regulated. Chemokine receptor expression also changes dramatically during the maturation process. For instance, CCR1, CCR5 are down-regulated sharply in mature cells, while CCR7 is up-regulated and appears on the cell surface within a few hours after addition of MCM. Functionally, mature dendritic cells are no longer capable of efficiently taking up antigen, but gain the ability to stimulate the proliferation of naïve T cells and B cells. Mature dendritic cells also change their migratory behaviors; they no longer respond to ligands for CCR1, CCR2 and CCR5, such as MIP-1α, RANTES and MIP-1β. Instead, they respond to CCR7 ligands SLC and ELC.

MCM is prepared by as described by Romani *et al.* 1996, with minor modifications. Briefly, petri dishes (100 mm, Falcon) are coated with 5 ml of human Ig (10 mg/mL) for 30 min at 37˚C and washed with PBS 2-3 times immediately before use. $50 \times 10^6$ PBMC in 8 ml are layered onto human Ig-coated plates for 1-2 hours. Non-adherent cells are washed away and discarded. The adherent cells are incubated in fresh complete medium (RPMI + 10% normal human serum) at 37˚C, and the resulting media (MCM) is collected after 24 hours. The TNF-α concentration in the MCM is determined by the standard ELISA method (*e.g.*, using a TNF-α ELISA kit (R&D Systems, Minneapolis, MN)). The final TNF-α level in MCM is adjusted to 50 U/ml by mixing an appropriate amount of MCM with RPMI/10% fetal calf serum.

Suitable methods for neutrophil purification are known in the art. According to one suitable method, whole fresh blood (WB) is diluted 1:1 with 3% dextran in a 50 ml centrifuge tube and allowed to sediment for 30 - 45 minutes at room temperature. Twenty-five ml of WB plus 25 ml dextran results in approximately 35 ml of supernatant after 30 minutes sedimentation. The supernatant is layered over 12-15 ml Ficoll and centrifuged at 400 x g for 30-40 minutes at 18-20˚C. The plasma/platelet layer containing mononuclear cells and Ficoll-Paque are removed by aspiration. Neutrophils are found in the white layer above the erythrocyte (RBC) layer. (In some preparations, the neutrophil and erythrocyte layers are mixed. In these cases, RBCs are removed by hypotonic lysis: 12.5 ml of cold 0.2% NaCl is added to the neutrophils/RBC pellet while vortexing. 12.5 ml of cold 1.6% NaCl is immediately added while still vortexing. The cells are centrifuged at 60- 100 x g for 10 m and recovered. Ifnecessary the lysis step is repeated). The resulting neutrophils are >95% pure (with the eosinophis as the primary remaining cells).

## Prognostic assays

The diagnostic methods described herein can furthermore be utilized to identify subjects having or at risk of developing a disease or disorder associated with aberrant FPRL1 receptor or FPRL1 ligand expression or activity. For example, the described assays can be used to identify a subject having or at risk of developing a disorder such as a neurodegenerative disorder. Typically, a test sample is obtained from a subject and FPRL1 receptor or FPRL-1 ligand is detected or activity is assayed. For example, a test sample can be a biological fluid (*e.g.*, serum), cell sample, or tissue.

Prognostic assays can be used to determine whether a subject can be administered a modality (*e.g.*, an agonist, antagonist, peptidomimetic, protein, peptide, nucleic acid, small molecule, food, *etc.*) to treat a disease or disorder associated with aberrant FPRL1 receptor or FPRL1 ligand expression or activity. Such methods can be used to determine whether a subject can be effectively treated with an agent for a disorder. Also described herein are methods for determining whether a subject can be effectively treated with an agent for a disorder associated with aberrant FPRL1 receptor or FPRL1 ligand expression or activity. In such an assay, a test sample is obtained and SHAAGtide or nucleic acid is detected (*e.g.*, where the presence of SHAAGtide or nucleic acid is diagnostic for a subject that can be administered the agent to treat a disorder associated with aberrant FPRL1 receptor or FPRL1 ligand expression or activity).

The following examples are given to illustrate the invention and are not intended to be limiting.

EXAMPLES

*Example 1: CKβ8-1 (25-116), like other CKβ8 variants, stimulates intracellular calcium flux in CCR1 expressing cells.*

The human recombinant chemokines, leukotactin, three known CKβ8 variants CKβ8(1-99), CKβ8(25-99), CKβ8-1(1-116) and a novel $NH_2$-terminal truncated form of CKβ8-1 , CKβ8-1(25-116) were obtained from R&D Systems (Minneapolis, MN). CKβ8-1(25-116) was compared with the other three variants for the ability to elicit an intracellular calcium mobilization in stable human CCR1 transfected HEK239 cells. Human HEK293-CCR1 cells were prepared using Fugena 6 (Roche, IN) following the manufacturer's protocol. The HEK-293 cell lines were maintained in DMEM with 10% FBS supplemented with 800μg/ml G-418.

Stable expression of human chemokine receptor CCR1 in HEK293 cell was obtained as follows: full length cDNA encoding CCR1 was cloned by the polymerase chain reaction (PCR) from genomic DNA isolated from human peripheral blood cells. The PCR product was cloned into pcDNA3.1 (Invitrogen, Carlsbad, CA) using standard molecular cloning procedures and completely sequenced to confirm identity.

Two micrograms of the CCR1/pcDNA3.1 construct were used to transfect the HEK293 cells as follows. FuGENE: DNA complex was prepared by mixing 6.0 μl FuGENE 6 reagent (Roche Molecular Biochemicals, CA) and 2 μg CCR1/pcDNA3.1 in 100 μl of serum-free medium (Hyclone, CO). After incubating for 30 minutes at room temperature, the complex was added to a 60 mm culture plate containing 0.5 -1 X $10^6$ cells in 10 ml DMEM medium supplemented with 10 % FBS (Hyclone, CO). After mixing, the cells were returned to the incubator to culture at 37˚C for two days. At 48 hours post-transfection, Genetinin (G418) (Mediatech, Hemdon, VA) was added at a final concentration of 800 ug/ml. The cells were then plated in 96-well plates at a concentration of 20,000 cells/well. After 2-3 weeks under G418 selection, stable geneticin-resistant CCR1 expressing cells were assessed for their ability to mobilize calcium in response to MIP-1α at a concentration of 1-500 nM.

$Ca^{2+}$ mobilization responses were performed using the intracellular ratiometric fluorescent dye, Indo-1. Cells were loaded with Indo-1/AM (3 μM; Molecular Probes, Eugene, OR) in culture medium (45 min, 20˚C, $10^7$ cells/ml). After dye loading, cells were washed once with 10 ml PBS) and resuspended at $10^6$ cell/ml in HBSS containing 1% FBS. Cytosolic [$Ca^{2+}$] release was determined using excitation at 350 nm using a Photon Technology International fluorimeter (excitation at 350 nm, ratioed dual emission at 400 and 490 nm).

With HEKCCR1-293 transfectants, CKβB8-1(25-116) and the other CKβ8 variants induced a rapid calcium flux at 100 nM. The two truncated variants CKβ8(25-99) and CKβ8-1(25-116) induced a high calcium response, while the signals generated by variants CKβ8(25-99) and CKβ8-1(1-116) were lower. None of these chemokines induced a signal with the untransfected parental HEK293 cells, demonstrating that the activity is due to CCR1 and not an endogenous receptor. The maximal receptor stimulations obtained with 100nM CKβ8(25-99) and CKβ8-1(25-116) were equivalent to those obtained with the same concentration of the CCR1 agonist, leukotactin.

*Example 2: CCL23 variant CKβ8-1(25-116) displays an unique activity profile in human monocyte and neutrophils that is not a CCR1 linked event.*

The human recombinant chemokines, leukotactin, MIP-1α, three known CKβ8 variants CKβ38(1-99), CKβ8 (25-99), CKβ8-1(1-116) and a novel $NH_2$-terminal truncated form of CKβ8-1, CKβ8-1(25-116) were obtained from R&D Systems (Minneapolis, MN). Human monocytes were generated from buffy coats (Stanford Blood Center, Palo Alto, CA) following a standard protocol. Briefly, PBMC were isolated by standard density gradient centrifugation (Ficoll-Paque-Plus, Pharmacia). Monocytes were purified using CD14 Microbeads (Miltenyi, Auburn, CA) magnetic positive selection. Human neutrophils were isolated from fresh peripheral blood from healthy individuals by gradient centrifugation on Ficoll-Hypaque (Hyclone, CA).

The activity of the CCL23 variants was tested on freshly prepared human monocytes and neutrophils using the calcium flux test described in Example 1. Although all of the chemokines stimulated some calcium release on monocytes, CKβ8(1-99) and CKβ8-1(1-116) showed poor activity, even at 250nM. CKβ8(25-99) showed slightly higher calcium stimulation. However, CKβ8-1(25-116) exhibited a unique calcium flux with extended calcium release. The maximal receptor stimulation obtained with 100 nM CKβ8-1(25-116) was at least two fold higher than that obtained with the same concentration of leukotactin.

On neutrophils, 100 nM leukotactin induced a calcium flux but neither MIP-1α nor CKβ8(1-99), CKβ8(25-99) or CKβ8(1-116) induced a calcium flux. However, CKβ8-1(25-116) induced an unique calcium release. The magnitude was much higher than observed for the same amount of leukotactin stimulation.

*Example 3: Cross-desensitization test performed on HEK293-CCR1 transfectants, monocytes and neutrophils.*

In cross-desensitization tests tests, cells were stimulated sequentially with leukotactin and then the chemokines CKβ8(1-99), CKβ8(25-99), CKβ8-1(1-116), and CKβ8-1(25-116). On HEK293-CCR1 transfectants (prepared as in Example 1), leukotactin induced similar patterns of receptor desensitization to all variants. When the cells were pretreated with 100 nM leukotactin, the calcium flux response to all ligands was completely inhibited.

Similar receptor cross-desensitization tests were performed using both monocytes and neutrophils (prepared as in Example 2). On monocytes, leukotactin completely desensitized the CCL23 variants, CKβ8(1-99), CKβ8(25-99), CKβ8-1(1-116). In contrast, leukotactin prestimulation did not desensitize CKβ8-1(25-116) activity on monocytes. On neutrophils, CKβ8(1-99), CKβ8(25-99), and CKβ8-1(1-116) were inactive and prestimulation with leukotactin had no effect. However, leukotactin prestimulation did not desensitize the stimulation with CKβ8-1(25-116).

*Example 4: CCL23 variants compete with $^{125}$I -MIP-1α for binding to CCR1-expressing cells.*

The binding characteristics of CCL23 variants was compared in human CCR1 expressing cells. The ability of MIP-1α and the CCL23 variants CKβ8(1-99), CKβ8(25-99), CKβ8-1(1-116) and CKβ8-1(25-116) to compete with $^{125}$I-MIP-1α binding was investigated in HEK293:CCR1 cells (prepared as in Example 1). The cells were incubated with $^{125}$I -labeled MIP-1α (final conc. ~ 0.05 nM) in the presence of unlabeled chemokine (3 hours at 4°C: 25 mM HEPES, 140 mM NaCl, I mM $CaCl_2$, 5mM $MgCl_2$ and 0.2% BSA, adjusted to pH 7.1). Reactions mixtures were aspirated onto PEI-treated GF/B glass filters using a cell harvester (Packard). The filters were washed twice (25 mM HEPES, 500 mM NaCl, 1 mM $CaCl_2$, 5 mM $MgCl_2$, adjusted to pH 7.1). Scintillant (MicroScint-10; 35 μl) was added to dried filters and the filiters counted in a Packard Topcount scintillation counter. Data were analyzed and plotted using Prism software (GraphPad Software, San Diego, CA).

Competition curves were observed with increasing concentrations of MIP-1α or CCL23 variants. MIP-1α gave an IC50 of 0.54 nM. The CCL23 variants gave IC50 values of 64 nM, 1.34 nM, 206 nM, and 112 nM, respectively. CKβ8-1 (1-116) showed 3-4 fold less potency than CKβ8(1-99) on this transfectant for the displacement of the bound $^{125}$I-MIP-1α from CCR1, consistent with its relatively weak affinity for CCR1. Also as expected, the truncation of CKβ8(1-99), CKβ8(25-99), showed a 40-fold IC50 increase. However, the IC50 for CKβ8-1(25-116), the same amino acid truncated variant of CKβ8-1(1-116), is only increased one fold.

Simiar binding competition tests were conducted on monocytes and neutrophils. These cells were prepared as in Example 2. Binding competition between MIP-1α on neutrophils could not be studied, since MIP-1α does not bind neutrophils. On monocytes, MIP-1α has an IC50 of 0.27 nM, and CCL23 variants IC50s of 10 nM, 0.25 nM, 55 nM, and 5 nM, respectively. Overall, CKβ8(1-99) and CKβ8(25-99) showed similar IC50 to that observed on HEK293-CCR1 cells. However, CKβ8-1(1-116) and CKβ8-1(25-116) showed higher MIP-1α displacement activities on monocytes, especially CKβ8-1(25-116) which was over 10 fold higher. $^{125}$I-MIP-1α binding-competition data (IC50) is shown in Table 6. The IC50 for each interaction was derived from non-linear least squares curve fitting.

Table 6. $^{125}$I-MIP-1α Binding Competition Data for HEK293-CCR1 Transfectants and Monocytes

|  | HEK293-CCR1 | Monocytes |
| --- | --- | --- |
| MIP1α | 0.54 nM | 0.27 nM |
| CKβ8(1-99) | 64 nM | 10.3 nM |
| CKβ8(25-99) | 1.34 nM | 0.25 nM |

(continued)

|  | HEK293-CCR1 | Monocytes |
|---|---|---|
| CKp8-1(1-116) | 206 nM | 55 nM |
| CKβ8-1(25-116) | 112 nM | 5.1 nM |

*Example 5 Variant CKβ8-1(25-116) induces human monocyte and neutrophil migration with a novel migratory property.*

Migration assays were performed on monocytes and neutrophils. Human monocytes and neutrophils (prepared as in Exampe 2) were harvested and resuspended in chemotaxis medium (CM). The CM consisted of Hank's buffered salt solution (Gibco, MA) containing $CaCl_2$ (1 mM) and MgSO4 (1 mM) with added 0.1% BSA (Sigma, St. Louis, MO). The assays were performed in 96-well ChemoTx® microplates (Neuroprobe, MA). Leukotactin, MIP-1α and chemokines (CKβ8(1-99), CKβ8(25-99), CKβ8-1(1-116) or CKβ8-1(25-116), prepared as in Example 1) were added to the lower wells (final volume 29 μL), and 20 μL of cell suspension ($5 \times 10^6$ cells/mL for monocytes; $2.5X 10^6$ cells/mL for neutrophils) added to the polycarbonate filters (5 μm pore size for monocytes; 3 μm pore size for neutrophils). After incubation for 90 min (37˚C, 100% humidity, 5 % $CO_2$), cells were removed from the upper surface of the filter by scraping. Cells that migrated into the lower chamber were quantified using the Quant cell proliferation assay kit (Molecular Probes, OR).

On monocytes, CKβ8(25-99), CKβ8(1-99) and CKβ8-1(1-116) showed moderate activity at all concentrations up to 100 nM. CKβ8-1(25-116) showed a dramatically higher activity than the other three variants at 100 nM, although its activity at and 1 and 10 nM was very similar to the other variants.

The same test was performed on human neutrophils. Human neutrophils generally lacked robust response to CCR1 ligands. Consistent with the calcium flux results, none of the known CCR1 ligands including leukotactin and M1P-1α was active. However, CKβ8-1(25-116) induced a robust response at 100 nM. This magnitude is comparable to many other potent CXCR1 and CXCR1 ligands including IL-8 and GRO-α.

*Example 6 CKβ8-1(25-116) is able to induce intracellular calcium flux and chemotaxis in formyl peptide receptor like 1 (FPRL1) expressing cells.*

The functional activities of CKβ8- 1(25-116) were investigated in human FPRL1-L1.2 transfectants and in native L1.2 cells. Stable expression of formyl peptide-like receptor 1 (FPRL1) in L1.2 cell was obtained as follows. Full length cDNA encoding FPRL1 was cloned, using the polymerase chain reaction (PCR), from genomic DNA isolated from undifferentiated HL-60 cells. The polymerase chain reaction product was cloned into pcDNA3.1 (Invitrogen, Carlsbad, CA) using standard molecular cloning procedures and completely sequenced to confirm identity. Twenty micrograms of the FPRL1/pcDNA3.1 construct were linearized by digestion with Bsm 1 (New England Biolabs, Beverly, MA) and used to transfect the murine B cell line L1.2 as follows. Twenty five million cells were washed twice and resuspended in 0.8 ml of PBS. The cells were incubated for 10 min at room temperature with the linearized FPRL/pcDNA3.1 construct DNA and transferred to a 0.4-cm cuvette, and a single electroporation pulse was applied at 250 V, 960 μF. Electroporated cells were incubated for 10 min at room temperature and transferred to culture at 37˚C in RPMI supplemented with 10% FCS. Geneticin (G418) was added to a final concentration of 800 μg/ml 48 h posttransfection and the cells plated into 96-well plates at 25,000 cells/well. After 2-3 weeks under drug selection, stable geneticin-resistant FPLR1 expressing cells were assessed for their ability to mobilize $Ca^{++}$ in response to SHAAGtide or CKbeta 8-1 at concentrations of l to 1000 nM.

A calcium flux test was performed on these transfectants using the method described-in Example 1. Of the CCL23 variants, only CKβ8-1(25-116) stimulated calcium release in FPRL1 expressing cells. The synthetic peptides Trp-Lys-Tyr-Met-Val-D-Met-NH$_2$ (WKYMVm) and Trp-Lys-Tyr-Met-Val-Met-NH$_2$ (WKYMVM) ("W peptides 1 and 2") (obtained from Phoenix Pharmaceuticals (Belmont, CA)), known non-natural ligands for FPRL1, produced a robust calcium flux. A CKβ8-1(25-116) induced calcium release was not observed in pariental cells or cells transfected with other chemokine receptors. When a CKβ8-1(25-116) induced calcium flux dose response assay was performed, an EC50 of 10-20 nM was observed on these cells. CKβ8-1(1-116) showed no activity on FPRL1 expressing cells, even at 200 nM.

The ability of CKβ8-1(25-116) to elicit the migration of the FPRL1 expressing cells was examined. Test conditions were as in Example 5. Although pariental L1.2 cells did not migrate in the assay, cells expressing FPRL1 migrated in a bell-shaped dose-dependent manner in response to CKβ8-1(25-116) concentrations ranging from 1 nM to 1 μM. The half-maximal cell migration was observed at 30 nM. The magnitude of the maximal response was higher than observed with the synthetic peptides WKYMVm and WKYMVM. In general, compared to the other chemokines, CKβ8-1(25-116) showed a broader bell-shaped curve in FPRL1 mediated migration. Hence, in addition to its activity on CCR1, CKβ8-1 (25-116) also functions through the receptor FPRL1 expressed on monocytes and neutrophils.

*Example 7 CKβ8-1(25-116) is able to displace* $^{125}$*I-labeled WKYMVm binding on human monocytes and FPRL1 expressing cells.*

The binding of CKβ8-1(25-116) to FPRL1 was determined by measuring the ability of CKβ8-1(25-116) to displace $^{125}$I--labeled WKYMVm($^{125}$I-labeled Trp-Lys-Tyr-Met-Val-D-Met-NH$_2$, Perkin Elmer Life Science (Boston, MA)) from human FPRL1-L1.2 transfectants and human monocytes. Cells were incubated with 0.01 nM $^{125}$I -WKYMVm in the presence of increasing concentrations of unlabeled WKYMVm or CKβ8-1(25-116) for three hours at 4 degree C. The IC50 for each interaction was derived from non-linear least squares curve fitting of the data by using Prism software (GraphPad Software).

For FPRL1-L1.2 transfectants and monocytes, competition curves were observed with increasing concentrations of WKYMVm or CKβ8-1(25-116) (Table 7). Such curves were not observed for other CCL23 variants.

Table 7 $^{125}$I-labeled WKYMVm competition curves observed with WKYMVm and CKβ8-1(25-116).

|  | IC50 | |
| --- | --- | --- |
|  | Human Monocytes | L1.2 EPRL1 Cells |
| WKYMVM | 1.5nM | 80nM |
| CKβ8-1(25-116) | 31nM | 196nM |

*Example 8 Chemokine or SHAAGtide induced calcium mobilization by Immature Dendritic Cells, Mature Dendritic Cells, Monocytes or Neutrophils.*

Human recombinant CKβ8-1 (25-116) chemokine was obtained from R&D Systems (Minneapolis, MN). The peptide SHAAGtides SEQ ID NO:1 and SEQ ID NO:6 and a control protein (the reverse sequence of SEQ ID NO:1) were synthesized and HPLC-purified using routine techniques as described in Sambrook *et al.,* 1989, and Ausubel *et al.,* 1999.

Human monocytes were either generated from buffy coats (Stanford Blood Center, Palo Also, CA) or from fresh blood of healthy individuals following a standard protocol. Briefly, PBMC were isolated by a Ficoll-Paque gradient centrifugation (Ficoll-Paque-Plus, Pharmacia). Monocytes were purified by CD14 Microbeads (Miltenyi) magnetic positive selection. Human neutrophils were isolated from fresh blood by dextran sedimentation and gradient centrifugation Ficoll-Paque gradient centrifugation. All cells were washed and resuspended ($1 \times 10^7$/ml) in RPMI medium with 10% FBS.

Immature DCs were derived by culturing CD14+ monocytes in the presence of GM-CSF and IL4. Briefly, monocytes were cultured in a T-175 flask at $10^6$ cells/ml in RPMI/10% FCS. Recombinant human GM-CSF and IL4 were added on day 0, 2, 4 and 6 to a final concentration of 1000 u/ml and 500 u/ml, respectively. Cells were harvested on day 7 as immature DCs and characterized for surface protein expression by FACS analysis. DC maturation was carried out by culturing day 6 immature DCs in macrophage-conditioned medium (MCM). Briefly, day 6 immature DCs were harvested by centrifugation and resuspended in MCM at $10^6$ cells/ml. The medium was supplemented with 1000 u/ml ofGM-CSF and 500 u/ml IL4. After three more days of culture, cells were harvested as mature DCs and characterized by surface protein expression flow cytometry.

MCM was prepared as follows: PBMCs isolated from buffy coat were incubated at 37oC in a plastic flask pre-coated with 10 mg/ml human IgG (Sigma, St Louis, MO) for 30 minutes. After 30 minutes, non-adherent cells were removed and adherent cells were washed three times with DPBS, then cultured in RPMI/10% human serum. Conditioned-medium was collected after 24 hours. TNF-α concentration, which is critical for DC maturation, was determined by using a TNF-α ELISA kit (R&D Systems, MN). The final TNF-α level in MCM was adjusted to 50 u/ml by mixing with RPMI/10% human serum, and was stored at-80 freezer until use.

Ca$^{2+}$ mobilization responses were performed using an intracellular ratiometric fluorescent dye, Indo-1. Cells were loaded with Indo-1/AM (3 μM; Molecular Probes (Eugene, OR)) in culture medium (45 min, 20˚C, $10^7$ cells/ml). After dye loading, cells were washed once (10 ml PBS) and resuspended at $10^6$ cell/ml in HBSS containing 1% FBS. Cytosolic [Ca$^{2+}$] release was determined using excitation at 350 nm using a Photon Technology International fluorimeter (excitation at 350 nm, ratioed dual emission at 400 and 490 nm).

The SHAAGtides SEQ ID NO:1 and SEQ ID NO:6, as well as CKβ8-1(25-116), produced a robust calcium flux on human monocytes and neutrophils and were partially active on immature Dendritic Cells and mature Dendritic Cells. No significant calcium flux was observed with the control peptide. Since immature Dendritic Cells express high levels of CCR1, CKβ8-1(25-116) induces Ca$^{2+}$ release in these cells.

*Example 9 Chemokine, SHAAGtide, and SHAAGtide truncated variants induced calcium mobilization by stable expressed FPRL1 cells.*

Stable expression of human FPRL1 in L1.2 cells was obtained as in Example 6. Calcium flux tests were conducted on the transfectants using the method described in Example 1. Chemokines (CKβ8(1-99), CKβ8(25-99), CKβ8-1(1-116) and CKβ8-1(25-116), were prepared as in Example 1. W peptides 1 and 2 were obtained as in Example 6. In addition, and the following SHAAGtide sequences and truncated variants (prepared as in Example 8) were tested:

|          |              |
|----------|--------------|
| CCXP1    | SEQ ID NO:1  |
| CCXP2    | SEQ ID NO:2  |
| CCXP3    | SEQ ID NO:3  |
| CCXP4    | SEQ ID NO:4  |
| CCXP5    | SEQ ID NO:5  |
| CCXP6    | SEQ ID NO:6  |
| CCXP7    | SEQ ID NO:7  |
| CCXP8    | SEQ ID NO:8  |
| CCXP9    | SEQ ID NO:9  |
| CCXP10   | SEQ ID NO:10 |

All ligands were added in a dose response manner and the peak calcium flux response determined. Table 8 shows that CKβ8(25-116) (SEQ ID NO:16) and certain SHAAGtides induced calcium mobilization in FPRL1 transfectants. However, CKβ8(1-116), which does not contain a free SHAAGtide N-terminal, did not give significant mobilization. The data also indicates that the N-terminal of the SHAAGtide is important for its activity in FPRL1 transfactants. Those SHAAGtides having a truncated N-terminal gave greatly reduced calcium mobilization. SHAAGtides having a truncated or substituted C-terminal did not exhibit the same loss in activity as was observed after truncation of the N-terminal.

Table 8 - Induction of Calcium Flux in FPRL1 L1.2 Cells. (IC50 - were no IC50 value is listed, the sequence showed low or no significant activity.)

|                              | IC 50        |
|------------------------------|--------------|
| SEQ ID NO:1                  | 150nM        |
| SEQ ID NO:2                  | >50μM        |
| SEQ ID NO:3                  | 68nM         |
| SEQ ID NO:4                  | -            |
| SEQ ID NO:5                  | 7.4nM        |
| SEQ ID NO:6                  | 38nM         |
| SEQ ID NO:7                  | -            |
| SEQ ID NO:8                  | 45nM         |
| SEQ ID NO:9                  | -            |
| SEQ ID NO:10                 | -            |
| SEQ ID NO:13 - CKβ8(1-99)    | -            |
| SEQ ID NO:14 - CKβ8(25-99)   | -            |
| SEQ ID NO:15 CKβ8(1-116)     | -            |
| SEQ ID NO:16 CKβ8(25-116)    | 11nM         |
| W peptide 1                  | 0.7nM        |
| W peptide 2                  | <0.1μM       |

*Example 10 Chemotactic activity of Chemokines, SHAAGtide and SHAAGtide truncated variants on FPRL1-L1.2 cells.*

The chemotactic activity of chemokines (CKβ8(1-99) and CKβ8(25-99), SHAAGtides (SEQ ID NO:1 and SEQ ID NO:2) and W peptides 1 and 2 (obtained as in Example 6) on FPRL1-L1.2 cells was determined in migration assays. The chemokines were prepared as in Example 1. The SHAAGtide sequences SEQ ID NO:1 and SEQ ID NO:2 were prepared as in Example 8. FPRL1-L1.2 cells were prepared as in Example 6.

The migration assays were performed in 96-well ChemoTx® microplates (Neuroprobe) using the protocol described in Example 5. Both SEQ ID NO:1 and CKβ8-1(25-116) migrated the transfectants, indicating that they are functional for this receptor.

*Example 11 Chemotactic activity of Chemokines, SHAAGtide and SHAAGtide truncated variants on human monocytes and neutrophils.*

The chemotactic activity on human monocytes and neutrophils of chemokines: CKβ8(1-99), CKβ8(25-99), CKβ8(1-116) and CKβ8(25-116); W peptides 1 and 2 and the SHAAGtides sequences SEQ ID NO:1 and SEQ ID NO: 2 was determined in migration assays. The above peptides were prepared as in previous examples. The migration assays were performed in 96-well CHEMOTX® microplates (Neuroprobe) using the protocol described in Example 5. Both SHAAGtide SEQ ID NO:1 and CKb8-1(25-116) produced migration of both neutrophils and monocytes.

**References**

US Patent No. 4,522,811. Serial injection of muramyldipeptides and liposomes enhances the anti-infective activity of muramyldipeptides. 1985

US Patent No. 5,223,409. Directed evolution of novel binding proteins. 1993.

US Patent No. 5,328,470. Treatment of diseases by site-specific instillation of cells or site-specific transformation of cells and kits therefor. 1994.

U.S. Pat. No. 5,994,126. Method for in vitro proliferation of dendritic cell precursors and heir use to produce immunogens. 1999.

Ausubel, F.M., R. Brent, R.E. Kingston, D.D. Moore, et al. 1987. Current protocols in molecular biology. John. Wiley & Sons, New York.

Baek SH, Seo JK, Chae CB, Suh PG, Ryu SH. 1996. Identification of the peptides that stimulate the phosphoinositide hydrolysis in lymphocyte cell lines from peptide libraries. J Biol Chem 271(14):8170-5.

Baggiolini, M., and C. A. Dahinden. 1994. CC chemokines in allergic inflammation. Immunol Today 15:127.

Baggiolini, M., B. Dewald, and B. Moser. 1997. Human chemokines: an update. Annu Rev Immunol 15:675.

Bender et al., 1996, J. Immunol. Methods 196:121-35

Berkhout, T. A., J. Gohil, P. Gonzalez, C. L. Nicols, K. E. Moores, C. H. Macphee, J. R. White, and P. H. Groot. 2000. Selective binding of the truncated form of the chemokine CKbeta8 (25- 99) to CC chemokine receptor I (CCR1). Biochem Pharmacol 59:591.

Bonecchi, R., N. Polentarutti, W. Luini, A. Borsatti, S. Bernasconi, M. Locati, C. Power, A. Proudfoot, T. N. Wells, C. Mackay, A. Mantovani, and S. Sozzani. 1999. Up-regulation of CCR1 and CCR3 and induction of chemotaxis to CC chemokines by IFN-gamma in human neutrophils. J Immuno/ 162:474.

Bao L, Gerard NP, Eddy RL Jr, Shows TB, Gerard C. 1992. Mapping of genes for the human C5a receptor (C5AR), human FMLP receptor (FPR), and two FMLP receptor homologue orphan receptors (FPRH1, FPRH2) to chromosome 19. Genomics 13(2):437-40.

Campbell et al., 1998, J Cell Biol 141:1053

Carell, T., E.A. Wintner, and J. Rebek Jr. 1994a. A novel procedure for the synthesis of libraries containing small organic molecules. Angewandte Chemie International Edition. 33:2059-2061.

Carell, T., E.A. Wintner, and J. Rebek Jr. 1994b. A solution phase screening procedure for the isolation of active compounds from a molecular library. Angewandte Chemie International Edition. 33:2061-2064.

Carter, P. 1986. Site-directed mutagenesis. Biochem J. 237:1-7.

Cella et al, 1999, J Exp Med. 189:821-9.

Chan et al., 1999, Blood 93:3610.

Chen, S.H., H.D. Shine, J.C. Goodman, R.G. Grossman, et al. 1994. Gene therapy for brain tumors: regression of experimental gliomas by adenovirus-mediated gene transfer in vivo. Proc Natl Acad Sci USA. 91:3054-7.

Cho, C.Y., E.J. Moran, S.R. Cherry, J.C. Stephans, et al. 1993. An unnatural biopolymer. Science. 261:1303-5.

Christophe, T., Karlsson, A., Dugave, C, Marie-Josèphe Rabiet, Francois Boulay, F., and Dahlgren, C. (2001). The Synthetic Peptide Trp-Lys-Tyr-Met-Val-Met-NH2 Specifically Activates Neutrophils through FPRL1/Lipoxin A4 Receptors and Is an Agonist for the Orphan Monocyte-expressed Chemoattractant Receptor FPRL2. J. Biol. Chem., Viol. 276, Issue 24, 21585-21593. Cohen. 1993. Science 259:1691-1692.

Coligan, 1991. Current Protocols in Immunology, Wiley/Greene, NY.

Cull, M.G., J.F. Miller, and P.J. Schatz. 1992. Screening for receptor ligands using large libraries of peptides linked to the C terminus of the lac repressor. Proc Natl Acad Sci USA. 89:1865-9.

Cwirla, S.E., E.A. Peters, R.W. Barrett, and W.J. Dower. 1990. Peptides on phage: a vast library of peptides for identifying ligands. Proc Natl Acad Sci USA. 87:6378-82.

Deng, X., Ueda, H., Su, S. B., Gong, W., Dunlop, N. M., Gao, J.-L., Murphy, P. M., and Wang, J. M. (1999) A Synthetic Peptide Derived From Human Immunodeficiency Virus Type 1 gp120 Downregulates the Expression and Function of Chemokine Receptors CCR5 and CXCR4 in Monocytes by Activating the 7-Transmembrane G-Protein-Coupled Receptor FPRL1/LXA4R. Blood 94, 1165-1173.

Devlin, J.J., L.C. Panganiban, and P.E. Devlin. 1990. Random peptide libraries: a source of specific protein binding molecules. Science. 249:404-6.

DeWitt, S.H., J.S. Kiely, C.J. Stankovic, M.C. Schroeder, et al. 1993. "Diversomers": an approach to nonpeptide, nonoligomeric chemical diversity. Proc Natl Acad Sci U S A. 90:6909-13.

Dieu et al., 1998, J Exp Med 188:373.

Fantuzzi, L., P. Borghi, V. Ciolli, G. Pavlakis, F. Belardelli, and S. Gessani. 1999. Loss of CCR2 expression and functional response to monocyte chemotactic protein (MCP-1) during the differentiation of human monocytes: role of secreted MCP-1 in the regulation of the chemotactic response. Blood 94:875 Felici, F., L. Castagnoli, A. Musacchio, R. Jappelli, et al. 1991. Selection of antibody ligands from a large library of oligopeptides expressed on a multivalent exposition vector. J Mol Biol. 222:301-10.

Fodor, S.P., R.P. Rava, X.C. Huang, A.C. Pease, et al. 1993. Multiplexed biochemical assays with biological chips. Nature. 364:555-6.

Forssmann, U., M. B. Delgado, M. Uguccioni, P. Loetscher, G. Garotta, and M. Baggiolini. 1997. CKbeta8, a novel CC chemokine that predominantly acts on monocytes. In FEBS Lett, Vol. 408, p. 211.

Gallop, M.A., R.W. Barrett, W.J. Dower, S.P. Fodor, et al. 1994. Applications of combinatorial technologies to drug discovery. 1. Background and peptide combinatorial libraries. J Med Chem. 37:1233-51.

Gennaro, A.R. 2000. Remington: The science and practice of pharmacy. Lippincott, Williams & Wilkins, Philadelphia, PA.

Harlow, E., and D. Lane. 1988. Antibodies: A laboratory manual. Cold Spring Harbor Laboratory Press, Cold Spring Harbor. 726 pp.

Harlow, E., and D. Lane. 1999. Using antibodies: A laboratory manual. Cold Spring Harbor Laboratory PRess, Cold Spring Harbor, New York.

Houghten, R.A., J.R. Appel, S.E. Blondelle, J.H. Cuervo, et al. 1992. The use of synthetic peptide combinatorial libraries for the identification of bioactive peptides. Biotechniques. 13:412-21.

Kaufman, R.J. 1990. Vectors used for expression in mammalian cells. Methods Enzymol. 185:487-511.

Kellermann et al., 1999, J Immunol 162:3859.

Kriegler, M. 1990. Gene transfer and expression: A laboratory manual. Stockton Press, New York. 242 pp

Lam, K.S. 1997. Application of combinatorial library methods in cancer research and drug discovery. Anticancer Drug Design. 12:145-167.

Lam, K.S., S.E. Salmon, E.M. Hersh, V.J. Hruby, et al. 1991. General method for rapid synthesis of multicomponent peptide mixtures. Nature. 354:82-84.

Le, Y., Oppenheim J. J., and Wang, J. M. 2001. Survey: Pleiotropic roles of formyl peptide receptors, Cytokine and Growth Factor Reviews 12: 91-105.

Lee, S. C., M. E. Brummet, S. Shahabuddin, T. G. Woodworth, S. N. Georas, K. M. Leiferman, S. C. Gilman, C. Stellato, R. P. Gladue, R. P. Schleimer, and L. A. Beck. 2000. Cutaneous injection of human subjects with macrophage inflammatory protein-1 alpha induces significant recruitment of neutrophils and monocytes. J Immunol 164: 3392

Luna, L. G. 1968. The Manual of Histologic Staining Methods of the Armed Forces Institute of Pathology", McGraw-Hill, 3rd edition.

Macphee, C. H., E. R. Appelbaum, K. Johanson, K. E. Moores, C. S. Imburgia, J. Fornwald, T. Berkhout, M. Brawner, P. H. Groot, K. O'Donnell, D. O'Shannessy, G. Scott, and J. R. White. 1998. Identification of a truncated form of the CC chemokine CK beta-8 demonstrating greatly enhanced biological activity. J Immunol 161:6273.

Mantovani, A. 1999. The chemokine system: redundancy for robust outputs. Immunol Today 20:254.

Murphy, P. M., Ozcelik, T., Kenney, R. T., Tiffany, H. L., McDermott, D., and Francke, U. 1992. A structural homologue of the N-formyl peptide receptor. Characterization and chromosome mapping of a peptide chemoattractant receptor family J. Biol. Chem. 267, 7637-7643

Murphy, P. M., M. Baggiolini, I. F. Charo, C. A. Hebert, R. Horuk, K. Matsushima, L. H. Miller, J. J. Oppenheim, and C. A. Power. 2000. International union of pharmacology. XXII. Nomenclature for chemokine receptors. Pharmacol Rev 52:145.

Patel, V. P., B. L. Kreider, Y. Li, H. Li, K. Leung, T. Salcedo, B. Nardelli, V. Pippalla, S. Gentz, R. Thotakura, D. Parmelee, R. Gentz, and G. Garotta. 1997. Molecular and functional characterization of two novel human C-C chemokines as inhibitors of two distinct classes of myeloid progenitors. J Exp Med 185:1163.

Romani et al., 1996, J. Immunol. Methods 196:137

Rollins, B. J. 1997. Chemokines. Blood 90:909.

Sambrook, J. 1989. Molecular cloning: a laboratory manual. Cold Spring Harbor Laboratory, Cold Spring Harbor.

Scott, J.K., and G.P. Smith. 1990. Searching for peptide ligands with an epitope library. Science. 249:386-90.

Shao Bo Su, Ji-liang Gao, Wang-hua Gong, Nancy M. Dunlop, Philip M. Murphy, Joost J. Oppenheim and Ji Ming Wang. 1999. T21/DP107, A Synthetic Leucine Zipper-Like Domain of the HIV-1 Envelope gp41, Attracts and Activates Human Phagocytes by Using G-Protein-Coupled Formyl Peptide Receptors. Journal of Immunology, 162: 5924-5930.

Su, S. B., Gao, J.-L., Gong, W., Dunlop, N. M., Murphy, P. M., Oppenheim, J. J., and Wang, J. M. 1999. T21/DP107, A Synthetic Leucine Zipper-Like Domain of the HIV-1 Envelope gp41, Attracts and Activates Human Phagocytes by Using G-Protein-Coupled Formyl Peptide Receptors. J. Immunol. 162, 5924-5930.

Uguccioni, M., M. D'Apuzzo, M. Loetscher, B. Dewald, and M. Baggiolini. 1995. Actions of the chemotactic cytokines MCP-1, MCP-2, MCP-3, RANTES, MIP-1 alpha and MIP-1 beta on human monocytes. Eur J Immunol 25:64.

Ulmer et al., (1993) Science 259:1745-1749

Verdijk et al., 1999, J Immunol. 1:57-61

Weber, C., K. U. Belge, P. von Hundelshausen, G. Draude, B. Steppich, M. Mack, M. Frankenberger, K. S. Weber, and H. W. Ziegler-Heitbrock. 2000. Differential chemokine receptor expression and function in human monocyte subpopulations. J Leukoc Biol 67:699.

Wells, J.A., M. Vasser, and D.B. Powers. 1985. Cassette mutagenesis: an efficient method for generation of multiple mutations at defined sites. Gene. 34:315-23. Ye, R. D., Cavanagh, S. L., Quehenberger, O., Prossnitz, E. R., and Cochrane, C. G. 1992. Isolation of a cDNA that encodes a novel granulocyte N-formyl peptide receptor. Biochem. Biophys. Res. Commun. 184, 582-589:

Youn, B. S., S. M. Zhang, H. E. Broxmeyer, S. Cooper, K. Antol, M. Fraser, Jr., and B. S. Kwon. 1998. Characterization of CKbeta8 and CKbeta8-1: two alternatively spliced forms of human beta-chemokine, chemoattractants for neutrophils, monocytes, and lymphocytes, and potent agonists at CC chemokine receptor 1. Blood 91:3118.

Zoller, M.J., and M. Smith. 1987. Oligonucleotide-directed mutagenesis: a simple method using two oligonucleotide primers and a single-stranded DNA template. Methods Enzymol. 154:329-50.

Zuckermann, R.N., E.J. Martin, D.C. Spellmeyer, G.B. Stauber, et al. 1994. Discovery of nanomolar ligands for 7-transmembrane G-protein-coupled receptors from a diverse N-(substituted)glycine peptoid library. J Med Chem. 37:2678-85.

**Claims**

1. An isolated protein or polypeptide which modulates FPRL1 receptor activity comprising a sequence at its N-terminal, the sequence having at least 90% identity to SEQ ID No. 1 over its full length, wherein the protein or polypeptide is not one of SEQ ID Nos. 15 and 16.

2. The isolated protein or polypeptide of claim 1, wherein said N-terminal sequence has at least 95% sequence identity to SEQ ID No. 1.

3. The isolated protein or polypeptide of claim 1, wherein the N-terminal sequence has 100% sequence identity to SEQ ID No. 1.

4. The isolated protein or polypeptide of claim 1, wherein said N-terminal sequence is SEQ ID No. 1 or SEQ ID No. 6.

5. An isolated protein or polypeptide comprising a sequence corresponding to SEQ ID NO. 3 at the N-terminal, wherein the polypeptide is not one of SEQ ID Nos. 15 and 16.

6. A kit comprising a pharmaceutical composition comprising the protein or polypeptide of any one of claims 1 to 5, a pharmaceutically acceptable carrier, and a syringe.

7. An isolated nucleic acid encoding the protein or polypeptide of claim 1.

8. The isolated nucleic acid of claim 7 comprising a nucleic acid sequence having at least 90% identity to SEQ ID No. 20.

9. The isolated nucleic acid of claim 7 comprising a nucleic acid sequence having at least 95% identity to SEQ ID No. 20.

10. The isolated nucleic acid of claim 7 comprising a nucleic acid sequence having at least 99% identity to SEQ ID No. 20.

11. An isolated nucleic acid having a sequence complementary to the nucleic acid of claim 10.

12. The isolated nucleic acid of claim 10 comprising SEQ ID No. 20 or SEQ ID No. 25.

13. A fusion protein comprising the protein or polypeptide of claim 1 fused to a protein or polypeptide which is not the protein or polypeptide of SEQ ID No. 1.

14. A method of identifying a modulator of binding of a protein or polypeptide comprising an N-terminal sequence, wherein the N-terminal sequence has at least 90% identity to SEQ ID No. 1, to a FPRL1 receptor, comprising:

contacting a cell expressing a FPRL1 receptor with a candidate compound in the presence of a labelled protein or polypeptide comprising a N-terminal sequence, wherein the N-terminal sequence has at least 90% identity to SEQ ID No. 1; and
comparing the level of binding of said protein or polypeptide with the FPRL1 receptor in the presence of the candidate compound with the level of binding of said protein or polypeptide with the FPRL1 receptor in the absence of the candidate compound,
wherein a decrease in binding indicates that the candidate compound is an inhibitor of binding, and an increase in binding indicates that the candidate compound is an enhancer of binding.

15. The method of claim 14, wherein the candidate compound is an antibody, peptide, nucleic acid or small molecule.

16. The method of claim 14, wherein the cell is a neutrophil, monocyte, T-lymphocyte or dendritic cell.

**Patentansprüche**

1. Isoliertes Protein oder Polypeptid, das eine FPRL1-Rezeptoraktivität moduliert, umfassend eine Sequenz an seinem N-Terminus, wobei die Sequenz mindestens 90% Identität zu SEQ ID Nr. 1 über ihre gesamte Länge aufweist, wobei das Protein oder Polypeptid keines der SEQ ID Nrn. 15 und 16 ist.

2. Isoliertes Protein oder Polypeptid nach Anspruch 1, wobei die N-terminale Sequenz mindestens 95% Sequenzidentität zu SEQ ID Nr. 1 aufweist.

3. Isoliertes Protein oder Polypeptid nach Anspruch 1, wobei die N-terminale Sequenz 100% Sequenzidentität zu SEQ ID Nr. 1 aufweist.

4. Isoliertes Protein oder Polypeptid nach Anspruch 1, wobei die N-terminale Sequenz SEQ ID Nr. 1 oder SEQ ID Nr. 6 ist.

5. Isoliertes Protein oder Polypeptid umfassend eine Sequenz, die SEQ ID Nr. 3 am N-Terminus entspricht, wobei das Polypeptid keine der SEQ ID Nrn. 15 und 16 ist.

6. Kit umfassend eine pharmazeutische Zusammensetzung umfassend das Protein oder Polypeptid nach einem der Ansprüche 1 bis 5, einen pharmazeutisch akzeptablen Träger, und eine Spritze.

7. Isolierte Nukleinsäure, die das Protein oder Polypeptid nach Anspruch 1 kodiert.

8. Isolierte Nukleinsäure nach Anspruch 7, umfassend eine Nukleinsäuresequenz, die mindestens 90% Identität zu SEQ ID Nr. 20 aufweist.

9. Isolierte Nukleinsäure nach Anspruch 7, umfassend eine Nukleinsäuresequenz, die mindestens 95% Identität zu SEQ ID Nr. 20 aufweist.

10. Isolierte Nukleinsäure nach Anspruch 7, umfassend eine Nukleinsäuresequenz, die mindestens 99% Identität zu SEQ ID Nr. 20 aufweist.

11. Isolierte Nukleinsäure, die eine Sequenz aufweist, die komplementär zu der Nukleinsäure nach Anspruch 10 ist.

12. Isolierte Nukleinsäure nach Anspruch 10, umfassend SEQ ID Nr. 20 oder SEQ ID Nr. 25.

13. Fusionsprotein umfassend das Protein oder Polypeptid nach Anspruch 1, fusioniert an ein Protein oder Polypeptid, das nicht das Protein oder Polypeptid der SEQ ID Nr. 1 ist.

14. Verfahren zur Identifizierung eines Modulators der Bindung eines Proteins oder Polypeptids umfassend eine N-terminale Sequenz, wobei die N-terminale Sequenz mindestens 90% Identität zu SEQ ID Nr. 1, zu einem FPRL1-Rezeptor aufweist, umfassend:

Inkontaktbringen einer Zelle, die einen FPRL1-Rezeptor exprimiert, mit einer in Frage kommenden Verbindung in der Anwesenheit eines markierten Proteins oder Polypeptids umfassend eine N-terminale Sequenz, wobei die N-terminale Sequenz mindestens 90% Identität zu SEQ ID Nr. 1 aufweist; und
Vergleichen des Levels an Bindung des Proteins oder Polypeptids mit dem FPRL1-Rezeptor in der Anwesenheit der in Frage kommenden Verbindung mit dem Level an Bindung des Proteins oder Polypeptids mit dem FPRL1-Rezeptor in der Abwesenheit der in Frage kommenden Verbindung,
wobei eine Abnahme der Bindung anzeigt, dass die in Frage kommende Verbindung ein Inhibitor der Bindung ist, und ein Anstieg der Bindung anzeigt, dass die in Frage kommende Verbindung ein Verstärker der Bindung ist.

15. Verfahren nach Anspruch 14, wobei die in Frage kommende Verbindung ein Antikörper, ein Peptid, eine Nukleinsäure oder ein kleines Molekül ist.

16. Verfahren nach Anspruch 14, wobei die Zelle ein Neutrophil, ein Monocyt, ein T-Lymphocyt oder eine dendritische Zelle ist.

**Revendications**

1. Protéine isolée ou polypeptide isolé qui module l'activité du récepteur FPRL1, comprenant une séquence à son extrémité N-terminale, la séquence présentant une identité d'au moins 90 % avec la SEQ ID N° 1 sur sa longueur totale, ladite protéine ou ledit polypeptide n'étant pas une des SEQ ID N°s 15 et 16.

2. Protéine isolée ou polypeptide isolé suivant la revendication 1, dans lequel ladite séquence N-terminale présente une identité de séquence d'au moins 95 % avec la SEQ ID N° 1.

3. Protéine isolée ou polypeptide isolé suivant la revendication 1, dans lequel la séquence N-terminale présente une identité de séquence de 100 % avec la SEQ ID N° 1.

4. Protéine isolée ou polypeptide isolé suivant la revendication 1, dans lequel ladite séquence N-terminale est la SEQ ID N° 1 ou la SEQ ID N° 6.

5. Protéine isolée ou polypeptide isolé comprenant une séquence correspondant à la SEQ ID N° 3 à l'extrémité N-terminale, ledit polypeptide n'étant pas une des SEQ ID N°s 15 et 16.

6. Kit comprenant une composition pharmaceutique qui comprend la protéine ou le polypeptide de l'une quelconque des revendications 1 à 5, un support pharmaceutiquement acceptable et une seringue.

7. Acide nucléique isolé codant pour la protéine ou le polypeptide de la revendication 1.

8. Acide nucléique isolé suivant la revendication 7, comprenant une séquence d'acide nucléique présentant une identité d'au moins 90 % avec la SEQ ID N° 20.

9. Acide nucléique isolé suivant la revendication 7, comprenant une séquence d'acide nucléique présentant une identité d'au moins 95 % avec la SEQ ID N° 20.

10. Acide nucléique isolé suivant la revendication 7, comprenant une séquence d'acide nucléique présentant une identité d'au moins 99 % avec la SEQ ID N° 20.

11. Acide nucléique isolé ayant une séquence complémentaire de l'acide nucléique de la revendication 10.

12. Acide nucléique isolé suivant la revendication 10, comprenant la SEQ ID N° 20 ou la SEQ ID N° 25.

13. Protéine de fusion comprenant la protéine ou le polypeptide de la revendication 1 fusionné avec une protéine ou un polypeptide qui n'est pas la protéine ou le polypeptide la SEQ ID N° 1.

14. Méthode pour identifier un modulateur de la liaison d'une protéine ou d'un polypeptide comprenant une séquence N-terminale, ladite séquence N-terminale présentant une identité d'au moins 90 % avec la SEQ ID N° 1, à un récepteur FPRL1, comprenant :

la mise en contact d'une cellule exprimant un récepteur FPRL1 avec un composé candidat en présence d'une protéine marquée ou d'un polypeptide marqué comprenant une séquence N-terminale, ladite séquence N-terminale présentant une identité d'au moins 90 % avec la SEQ ID N° 1 ; et
la comparaison du degré de liaison de ladite protéine ou dudit polypeptide avec le récepteur FPRL1 en présence du composé candidat avec le degré de liaison de ladite protéine ou dudit polypeptide avec le récepteur FPRL1 en l'absence du composé candidat,
dans laquelle une diminution de liaison indique que le composé candidat est un inhibiteur de la liaison et une augmentation de liaison indique que le composé candidat est un amplificateur de la liaison.

15. Méthode suivant la revendication 14, dans laquelle le composé candidat est un anticorps, un peptide, un acide nucléique ou une petite molécule.

16. Méthode suivant la revendication 14, dans laquelle la cellule est une cellule neutrophile, un monocyte, un lymphocyte T ou une cellule dendritique.

# Figure 1 *Reported FPRL1 ligands*

| Ligands | Calcium Flux (EC50) | Migration (EC50) | Binding (EC50) |
|---|---|---|---|
| *Endogenous Ligands:* | | | |
| Lipoxin A4 | ? | ? | 2-10 nM to $H^3$-LPX |
| Serum amyloid A | >0.2 uM | >0.2uM | 250 nM to $I^{125}$-SSA |
| β-Amyloid (1-42) | >2uM | 10 uM | |
| Prion Peptide PrP(106-126) | >2 uM | 25-50 uM | |
| LL-37[1] | >5 uM | 5-10 uM | |
| D2D3(88-274)[2] | ? | 0.1 nM (on 293-FPRL1)[3] | 83 nM to $I^{125}$-D2D3(88-274) |
| *Viral & Bacterial Encoded:* | | | |
| T21/DP107 (HIV gp41) | >0.5 uM | >0.5 uM | |
| HIV gp120(414-434) | 5-10 uM | 5-10 uM | |
| H. pylori peptide, Hp(2-20)[4] | 10 uM | | |
| fMLP | >5 uM | | |
| *Non-natural Ligands:* | | | |
| WKYMVm | 0.1-1 nM | 0.1-1 nM | 10 nM to $I^{125}$-W Peptide |
| WKYMVM | 1-10 nM | 1-10 nM | >10 nM to $I^{125}$-W Peptide |
| MMK-1 | low nM | low nM | |

*Notes:*

1. Human cathelicidin-derived antibacterial peptide, LL-37.
2. Urokinase plasminogen activator (uPA), D2D3(88-274).
3. In neutrophils, neither uPA nor D2D3(88-274) induce calcium flux.
4. Helicobacter pylori peptide, Hp(2-20).

EP 1 434 854 B1

| | 1 | 11 | 21 | 31 | 41 | 51 |

CKβ8 (1-99)      RVTKDAETEFMMSKLPLENPVLLD----------------RFHATSADCCISYTPRSIP
CKβ8 (25-99)                                          RFHATSADCCISYTPRSIP
CKβ8-1 (1-116)   RVTKDAETEFMMSKLPLENPVLLDMLWRRKIGPQMTLSHAAGFHATSADCCISYTPRSIP
CKβ8-1 (25-116)                  MLWRRKIGPQMTLSHAAGFHATSADCCISYTPRSIP
MIP1δ            QFINDAETELMMSKLPLENPVVLN----------------SFHF-AADCCTSYISQSIP
Leukotactin                                          SFHF-AADCCTSYISQSIP
MIP1α                                                SLAADTPTACCFSYTSRQIP

| | 61 | 71 | 81 | 91 | 88 | 88 |

CKβ8 (1-99)      CSLLESYFETNSECSKPGVIFLTKKGRRFCANPSDKQVQVCMRMLKLDTRIKTRKN
CKβ8 (25-99)     CSLLESYFETNSECSKPGVIFLTKKGRRFCANPSDKQVQVCMRMLKLDTRIKTRKN
CKβ8-1 (1-116)   CSLLESYFETNSECSKPGVIFLTKKGRRFCANPSDKQVQVCMRMLKLDTRIKTRKN
CKβ8-1 (25-116)  CSLLESYFETNSECSKPGVIFLTKKGRRFCANPSDKQVQVCMRMLKLDTRIKTRKN
MIP1δ            CSLMKSYFETSSECSKPGVIFLTKKGRQVCAKPSGPGVQDCMKKLKPYSI
Leukotactin      CSLMKSYFETSSECSKPGVIFLTKKGRQVCAKPSGPGVQDCMKKLKPYSI
MIP1α            QNFIADYFETSSQCSKPGVIFLTKRSRQVCADPSEEWVQKYVSDLE

## Figure 2.

C Kβ8(1-99)     SEQ ID NO:13          MIP-1δ        SEQ ID NO:17
C Kβ8(25-99)    SEQ ID NO:14          Leukotactin   SEQ ID NO:18
C Kβ8(1-116)    SEQ ID NO:15          MIP-1α        SEQ ID NO:19
C Kβ8(25-116)   SEQ ID NO:16

## REFERENCES CITED IN THE DESCRIPTION

### Patent documents cited in the description

- US 4522811 A, Eppstein
- US 5328470 A, Nabel and Nabel
- US 5223409 A, Ladner
- US 09721902 B
- US 5994126 A

### Non-patent literature cited in the description

- **Kaufman.** *Methods Enzymol,* 1990, vol. 185, 487-511
- **Ausubel, F.M. ; R. Brent ; R.E. Kingston ; D.D. Moore et al.** Current protocols in molecular biology. John. Wiley & Sons, 1987
- **Baek SH ; Seo JK ; Chae CB ; Suh PG ; Ryu SH.** Identification of the peptides that stimulate the phosphoinositide hydrolysis in lymphocyte cell lines from peptide libraries. *J Biol Chem,* 1996, vol. 271 (14), 8170-5
- **Baggiolini, M. ; C. A. Dahinden.** CC chemokines in allergic inflammation. *Immunol Today,* 1994, vol. 15, 127
- **Baggiolini, M. ; B. Dewald ; B. Moser.** Human chemokines: an update. *Annu Rev Immunol,* 1997, vol. 15, 675
- **Bender et al.** *J. Immunol. Methods,* 1996, vol. 196, 121-35
- **Berkhout, T. A. ; J. Gohil ; P. Gonzalez ; C. L. Nicols ; K. E. Moores ; C. H. Macphee ; J. R. White ; P. H. Groot.** Selective binding of the truncated form of the chemokine CKbeta8 (25- 99) to CC chemokine receptor I (CCR1). *Biochem Pharmacol,* 2000, vol. 59, 591
- **Bonecchi, R. ; N. Polentarutti ; W. Luini ; A. Borsatti ; S. Bernasconi ; M. Locati ; C. Powe ; A. Proudfoot ; T. N. Wells ; C. Mackay.** Up-regulation of CCR1 and CCR3 and induction of chemotaxis to CC chemokines by IFN-gamma in human neutrophils. *J Immuno,* 1999, vol. 162, 474
- **Bao L ; Gerard NP ; Eddy RL Jr ; Shows TB ; Gerard C.** Mapping of genes for the human C5a receptor (C5AR), human FMLP receptor (FPR), and two FMLP receptor homologue orphan receptors (FPRH1, FPRH2) to chromosome 19. *Genomics,* 1998, vol. 13 ((2)), 437-40
- **Campbell et al.** *J Cell Biol,* 1998, vol. 141, 1053
- **Carell, T. ; E.A. Wintner ; J. Rebek Jr.** A novel procedure for the synthesis of libraries containing small organic molecules. *Angewandte Chemie International Edition.,* 1994, vol. 33, 2059-2061
- **Carell, T. ; E.A. Wintner ; J. Rebek Jr.** A solution phase screening procedure for the isolation of active compounds from a molecular library. *Angewandte Chemie International Edition.,* 1994, vol. 33, 2061-2064
- **Carter, P.** Site-directed mutagenesis. *Biochem J.,* 1986, vol. 237, 1-7
- **Cella et al.** *J Exp Med,* 1999, vol. 189, 821-9
- **Chan et al.** *Blood,* 1999, vol. 93, 3610
- **Chen, S.H. ; H.D. Shine ; J.C. Goodman ; R.G. Grossman et al.** Gene therapy for brain tumors: regression of experimental gliomas by adenovirus-mediated gene transfer in vivo. *Proc Natl Acad Sci USA.,* 1994, vol. 91, 3054-7
- **Cho, C.Y. ; E.J. Moran ; S.R. Cherry ; J.C. Stephans et al.** An unnatural biopolymer. *Science,* 1993, vol. 261, 1303-5
- **Christophe, T. ; Karlsson, A. ; Dugave, C ; Marie-Josèphe Rabiet ; Francois Boulay, F. ; Dahlgren, C.** The Synthetic Peptide Trp-Lys-Tyr-Met-Val-Met-NH2 Specifically Activates Neutrophils through FPRL1/Lipoxin A4 Receptors and Is an Agonist for the Orphan Monocyte-expressed Chemoattractant Receptor FPRL2. *J. Biol. Chem.,* 2001, vol. 276 (24), 21585-21593
- **Cohen.** *Science,* 1993, vol. 259, 1691-1692
- **Coligan.** Current Protocols in Immunology. Wiley/Greene, 1991
- **Cull, M.G. ; J.F. Miller ; P.J. Schatz.** Screening for receptor ligands using large libraries of peptides linked to the C terminus of the lac repressor. *Proc Natl Acad Sci USA.,* 1992, vol. 89, 1865-9
- **Cwirla, S.E. ; E.A. Peters ; R.W. Barrett ; W.J. Dower.** Peptides on phage: a vast library of peptides for identifying ligands. *Proc Natl Acad Sci USA.,* 1990, vol. 87, 6378-82

- **Deng, X. ; Ueda, H. ; Su, S. B. ; Gong, W. ; Dunlop, N. M. ; Gao, J.-L. ; Murphy, P. M. ; Wang, J. M.** A Synthetic Peptide Derived From Human Immunodeficiency Virus Type 1 gp120 Downregulates the Expression and Function of Chemokine Receptors CCR5 and CXCR4 in Monocytes by Activating the 7-Transmembrane G-Protein-Coupled Receptor FPRL1/LXA4R. *Blood,* 1999, vol. 94, 1165-1173
- **Devlin, J.J. ; L.C. Panganiban ; P.E. Devlin.** Random peptide libraries: a source of specific protein binding molecules. *Science,* 1990, vol. 249, 404-6
- **DeWitt, S.H. ; J.S. Kiely ; C.J. Stankovic ; M.C. Schroeder et al.** ''Diversomers'': an approach to nonpeptide, nonoligomeric chemical diversity. *Proc Natl Acad Sci U S A.,* 1993, vol. 90, 6909-13
- **Dieu et al.** *J Exp Med,* 1998, vol. 188, 373
- **Fantuzzi, L. ; P. Borghi ; V. Ciolli ; G. Pavlakis ; F. Belardelli ; S. Gessani.** Loss of CCR2 expression and functional response to monocyte chemotactic protein (MCP-1) during the differentiation of human monocytes: role of secreted MCP-1 in the regulation of the chemotactic response. *Blood,* 1999, vol. 94, 875
- **Felici, F. ; L. Castagnoli ; A. Musacchio ; R. Jappelli et al.** Selection of antibody ligands from a large library of oligopeptides expressed on a multivalent exposition vector. *J Mol Biol.,* 1991, vol. 222, 301-10
- **Fodor, S.P. ; R.P. Rava ; X.C. Huang ; A.C. Pease et al.** Multiplexed biochemical assays with biological chips. *Nature,* 1993, vol. 364, 555-6
- **Forssmann, U. ; M. B. Delgado ; M. Uguccioni ; P. Loetscher ; G. Garotta ; M. Baggiolini.** CKbeta8, a novel CC chemokine that predominantly acts on monocytes. *FEBS Lett,* 1997, vol. 408, 211
- **Gallop, M.A. ; R.W. Barrett ; W.J. Dower ; S.P. Fodor et al.** Applications of combinatorial technologies to drug discovery. 1. Background and peptide combinatorial libraries. *J Med Chem,* 1994, vol. 37, 1233-51
- **Gennaro, A.R.** Remington: The science and practice of pharmacy. Lippincott, Williams & Wilkins, 2000
- **Harlow, E. ; D. Lane.** Antibodies: A laboratory manual. Cold Spring Harbor Laboratory Press, 1988, 726
- **Harlow, E. ; D. Lane.** Using antibodies: A laboratory manual. Cold Spring Harbor Laboratory PRess, 1999
- **Houghten, R.A. ; J.R. Appel ; S.E. Blondelle ; J.H. Cuervo et al.** The use of synthetic peptide combinatorial libraries for the identification of bioactive peptides. *Biotechniques,* 1992, vol. 13, 412-21
- **Kaufman, R.J.** Vectors used for expression in mammalian cells. *Methods Enzymol.,* 1990, vol. 185, 487-511
- **Kellermann et al.** *J Immunol,* 1999, vol. 162, 3859
- **Kriegler, M.** Gene transfer and expression: A laboratory manual. Stockton Press, 1990, 242
- **Lam, K.S.** Application of combinatorial library methods in cancer research and drug discovery. *Anticancer Drug Design.,* 1997, vol. 12, 145-167
- **Lam, K.S. ; S.E. Salmon ; E.M. Hersh ; V.J. Hruby et al.** General method for rapid synthesis of multicomponent peptide mixtures. *Nature,* 1991, vol. 354, 82-84
- **Le, Y. ; Oppenheim J. J. ; Wang, J. M.** Survey: Pleiotropic roles of formyl peptide receptors. *Cytokine and Growth Factor Reviews,* 2001, vol. 12, 91-105
- **Lee, S. C. ; M. E. Brummet ; S. Shahabuddin ; T. G. Woodworth ; S. N. Georas ; K. M. Leiferman ; S. C. Gilman ; C. Stellato ; R. P. Gladue ; R. P. Schleimer.** Cutaneous injection of human subjects with macrophage inflammatory protein-1 alpha induces significant recruitment of neutrophils and monocytes. *J Immunol,* 2000, vol. 164, 3392
- **Luna, L. G.** The Manual of Histologic Staining Methods of the Armed Forces Institute of Pathology. McGraw-Hill, 1968
- **Macphee, C. H. ; E. R. Appelbaum ; K. Johanson ; K. E. Moores ; C. S. Imburgia ; J. Fornwald ; T. Berkhout ; M. Brawner ; P. H. Groot ; K. O'Donnell.** Identification of a truncated form of the CC chemokine CK beta-8 demonstrating greatly enhanced biological activity. *J Immunol,* 1998, vol. 161, 6273
- **Mantovani, A.** The chemokine system: redundancy for robust outputs. *Immunol Today,* 1999, vol. 20, 254
- **Murphy, P. M. ; Ozcelik, T. ; Kenney, R. T. ; Tiffany, H. L. ; McDermott, D. ; Francke, U.** A structural homologue of the N-formyl peptide receptor. Characterization and chromosome mapping of a peptide chemoattractant receptor family. *J. Biol. Chem.,* 1992, vol. 267, 7637-7643
- **Murphy, P. M. ; M. Baggiolini ; I. F. Charo ; C. A. Hebert ; R. Horuk ; K. Matsushima ; L. H. Miller ; J. J. Oppenheim ; C. A. Power.** International union of pharmacology. XXII. Nomenclature for chemokine receptors. *Pharmacol Rev,* 2000, vol. 52, 145
- **Patel, V. P. ; B. L. Kreider ; Y. Li ; H. Li ; K. Leung ; T. Salcedo ; B. Nardelli ; V. Pippalla ; S. Gentz ; R. Thotakura.** Molecular and functional characterization of two novel human C-C chemokines as inhibitors of two distinct classes of myeloid progenitors. *J Exp Med,* 1997, vol. 185, 1163
- **Romani et al.** *J. Immunol. Methods,* 1996, vol. 196, 137

- **Rollins, B. J.** *Chemokines. Blood,* vol. 90, 909
- **Sambrook, J.** Molecular cloning: a laboratory manual. Cold Spring Harbor Laboratory, 1989
- **Scott, J.K. ; G.P. Smith.** Searching for peptide ligands with an epitope library. *Science,* 1990, vol. 249, 386-90
- **Shao Bo Su ; Ji-liang Gao ; Wang-hua Gong ; Nancy M. Dunlop ; Philip M. Murphy ; Joost J. Oppenheim ; Ji Ming Wang.** T21/DP107, A Synthetic Leucine Zipper-Like Domain of the HIV-1 Envelope gp41, Attracts and Activates Human Phagocytes by Using G-Protein-Coupled Formyl Peptide Receptors. *Journal of Immunology,* 1999, vol. 162, 5924-5930
- **Su, S. B. ; Gao, J.-L. ; Gong, W. ; Dunlop, N. M. ; Murphy, P. M. ; Oppenheim, J. J. ; Wang, J. M.** T21/DP107, A Synthetic Leucine Zipper-Like Domain of the HIV-1 Envelope gp41, Attracts and Activates Human Phagocytes by Using G-Protein-Coupled Formyl Peptide Receptors. *J. Immunol.,* 1999, vol. 162, 5924-5930
- **Uguccioni, M. ; M. D'Apuzzo ; M. Loetscher ; B. Dewald ; M. Baggiolini.** Actions of the chemotactic cytokines MCP-1, MCP-2, MCP-3, RANTES, MIP-1 alpha and MIP-1 beta on human monocytes. *Eur J Immunol,* 1995, vol. 25, 64
- **Ulmer et al.** *Science,* 1993, vol. 259, 1745-1749
- **Verdijk et al.** *J Immunol.,* 1999, vol. 1, 57-61
- **Weber, C. ; K. U. Belge ; P. von Hundelshausen ; G. Draude ; B. Steppich ; M. Mack ; M. Frankenberger ; K. S. Weber ; H. W. Ziegler-Heitbrock.** Differential chemokine receptor expression and function in human monocyte subpopulations. *J Leukoc Biol,* 2000, vol. 67, 699
- **Wells, J.A. ; M. Vasser ; D.B. Powers.** Cassette mutagenesis: an efficient method for generation of multiple mutations at defined sites. *Gene,* 1985, vol. 34, 315-23
- **Ye, R. D. ; Cavanagh, S. L. ; Quehenberger, O. ; Prossnitz, E. R. ; Cochrane, C. G.** Isolation of a cDNA that encodes a novel granulocyte N-formyl peptide receptor. *Biochem. Biophys. Res. Commun.,* 1992, vol. 184, 582-589
- **Youn, B. S. ; S. M. Zhang ; H. E. Broxmeyer ; S. Cooper ; K. Antol ; M. Fraser, Jr. ; B. S. Kwon.** Characterization of CKbeta8 and CKbeta8-1: two alternatively spliced forms of human beta-chemokine, chemoattractants for neutrophils, monocytes, and lymphocytes, and potent agonists at CC chemokine receptor 1. *Blood,* 1998, vol. 91, 3118
- **Zoller, M.J. ; M. Smith.** Oligonucleotide-directed mutagenesis: a simple method using two oligonucleotide primers and a single-stranded DNA template. *Methods Enzymol.,* 1987, vol. 154, 329-50
- **Zuckermann, R.N. ; E.J. Martin ; D.C. Spellmeyer ; G.B. Stauber et al.** Discovery of nanomolar ligands for 7-transmembrane G-protein-coupled receptors from a diverse N-(substituted)glycine peptoid library. *J Med Chem.,* 1994, vol. 37, 2678-85